(19) Europäisches Patentamt
European Patent Office
Office européen des brevets

(11) **EP 2 515 878 B1**

(12) **EUROPEAN PATENT SPECIFICATION**

(45) Date of publication and mention
of the grant of the patent:
**30.10.2013 Bulletin 2013/44**

(21) Application number: **10805747.2**

(22) Date of filing: **21.12.2010**

(51) Int Cl.:
*A61K 9/16* (2006.01)    *A61K 38/00* (2006.01)
*A61K 9/00* (2006.01)    *A61P 35/00* (2006.01)

(86) International application number:
**PCT/JP2010/073665**

(87) International publication number:
**WO 2011/078394 (30.06.2011 Gazette 2011/26)**

(54) **SUSTAINED-RELEASE FORMULATION**

FORMULIERUNG MIT VERZÖGERTER FREISETZUNG

FORMULATION À LIBÉRATION PROLONGÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **22.12.2009  JP 2009290364**
**25.06.2010  JP 2010144793**

(43) Date of publication of application:
**31.10.2012  Bulletin 2012/44**

(73) Proprietor: **Takeda Pharmaceutical Company Limited**
**Osaka-shi, Osaka 541-0045 (JP)**

(72) Inventors:
• **FUTO, Tomomichi**
**Osaka-shi**
**Osaka 532-8686 (JP)**
• **TAIRA, Hikaru**
**Osaka-shi**
**Osaka 532-8686 (JP)**
• **MIZUKAMI, Seitaro**
**Osaka-shi**
**Osaka 532-8686 (JP)**
• **MURATA, Naoyuki**
**Osaka-shi**
**Osaka 532-8686 (JP)**

(74) Representative: **Kingsbury, Oliver William**
**Elkington and Fife LLP**
**Prospect House**
**8 Pembroke Road**
**GB-Sevenoaks, Kent TN13 1XR (GB)**

(56) References cited:
**WO-A1-2007/072997    WO-A1-2010/076896**
**JP-A- 2003 002 841**

SEQUENCE LISTING

<110> Takeda Pharmaceutical Company Limited

<120> Sustained-release formulation

<130> PCT10-0052

<150> JP2009-290364
<151> 2009-12-22

<150> JP2010-144793
<151> 2010-06-25

<160> 2

<170> PatentIn version 3.5

<210> 1
<211> 9
<212> PRT
<213> Artificial Sequence

<220>
<223> Synthetic peptide

<220>
<221> MISC_FEATURE
<222> (1)..(1)
<223> Xaa is Acetyl-D-Tyrosine.

<220>
<221> MISC_FEATURE
<222> (2)..(2)
<223> Xaa is trans-4-hydroxyproline.

<220>
<221> MISC_FEATURE
<222> (6)..(6)
<223> Xaa is Azaglycine.

<220>
<221> MISC_FEATURE
<222> (8).. (8)
<223> Xaa is N-omega-methylarginine.

<400> 1

```
                    Xaa Xaa Asn Thr Phe Xaa Leu Xaa Trp
                    1               5
```

<210> 2
<211> 9
<212> PRT
<213> Artificial Sequence

<220>
<223> Synthetic peptide

<220>
<221> MISC_FEATURE
<222> (1)..(1)
<223> Xaa is Acetyl-D-tyrosine.

<220>
<221> MISC_FEATURE

<222> (2)..(2)
<223> Xaa is D-tryptophan.

<220>
<221> MISC_FEATURE
<222> (6)..(6)
<223> Xaa is Azaglycine.

<220>
<221> MISC_FEATURE
<222> (8)..(8)
<223> Xaa is N-omega-methylarginine.

<400> 2

```
Xaa Xaa Asn Thr Phe Xaa Leu Xaa Trp
1               5
```

**Description**

## BACKGROUND OF THE INTENTION

**1. Field of the invention**

The present invention relates to a novel sustained-release formulation and the like which can effectively treat cancer and the like.

**2. Related Art**

As a stable metastin derivative having an excellent metastin-like activity, for example, a compound described in WO07/72997 is known. Furthermore, as a sustained-release formulation containing metastin or a derivative thereof for example, the formulation described in WO02/85399 is known.

To reduce a side effect by requiring no high dosage amount in order to obtain a medicinal effect, to improve patient's convenience and overcome pain by reducing the number of administration times and to obtain an effect of producing a medicinal effect for a long time, it is desired to develop a sustained-release formulation capable of slowly releasing a metastin derivative for a long time and having excellent properties as a clinical medicine.

## SUMMARY OF THE INVENTION

The present inventors conducted intensive studies with a view to solving the aforementioned problems. As a result, they found that a sustained-release formulation according to the present application containing a metastin derivative or a salt thereof and a lactic acid-glycolic acid copolymer having a weight average molecular weight of 5, 000 to 40, 000 or a salt thereof has excellent properties that are required for a clinical medicine in view of a medicinal effect, safety, stability, dosage amount, dosage form and use method, and then achieved the present invention.

More specifically, the present invention relates to the following sustained-release formulation (preparation) and a method for producing the same.

[1] A sustained-release formulation comprising a compound represented by Formula:

Ac-D-Tyr-Hyp-Asn-Thr-Phe-AzaGly-Leu-Arg(Me) -Trp-NH$_2$ (I)

(in the specification, sometimes simply referred to as compound (I)) or a salt thereof and a lactic acid-glycolic acid copolymer having a weight average molecular weight of 5,000 to  40,000 or a salt thereon,

[2] The sustained-release formulation according to item [1] above, wherein the weight average molecular weight of the lactic acid-glycolic acid copolymer is 6,000 to 20,000;

[3] The sustained-release formulation according to item [1] above, wherein the content of a glycolic acid of the lactic acid-glycolic acid copolymer or a salt thereof is greater than 0 wt% and 60 wt% or less;

[4] The sustained-release formulation according to item [1] above, wherein the content of a glycolic acid of the lactic acid-glycolic acid copolymer is 5 wt% or more and 50 wt% or less;

[5] The sustained-release formulation according to item [1] above, wherein the formulation is a therapeutic or prophylactic agent for cancer;

[6] The sustained-release formulation according to item [1] above, wherein the formulation is a parenteral agent;

[7] A method for producing the sustained-release formulation according to item [1] above, comprising producing a W/O emulsion composed of an internal water phase, which contains a compound represented by Formula:

Ac-D-Tyr-Hyp-Asn-Thr-Phe-AzaGly-Leu-Arg(Me) -Trp-NH$_2$ (I)

or a salt thereof, and an oil phase, which contains a lactic acid-glycolic acid copolymer or a salt thereof, further emulsifying the W/O emulsion to obtain a W/O/W emulsion, and subjecting the W/O/W emulsion to an in-water-drying method; and

[8] The method according to item [7] above, wherein the W/O emulsion is produced at a temperature of 31°C or more. Furthermore, the present invention also relates to a sustained-release formulation and therapeutic method according to the following aspects.

[9] A sustained-release formulation comprising compound (I) or a salt thereof and a lactic acid-glycolic acid copolymer having a weight average molecular weight of 5,000 to 40,000 or a salt thereof, which is used such that compound (I) or a salt thereof is administered to a patient in a dose of 0.01 1 to 4 mg/kg body weight at intervals of once 3 weeks or more (preferably, one month);

[10] The sustained-release formulation according to item [9] above produced by a method using a W/O/W emulsion;

[11] A sustained-release formulation comprising compound (I) or a salt thereof and a lactic acid-glycolic acid copolymer having a weight average molecular weight of 5,000 to 40,000 or a salt thereof which is used such that compound (I) or a salt thereof is administered to a patient in a dose of 0.09 to 1.8 mg/kg body weight at intervals of once per 12 weeks or more (preferably, 3 months);

[12] The sustained-release formulation according to any of items [1] to [6] and [9] to [11] above for use in treating or preventing cancer (for example, lung cancer, stomach cancer, liver cancer, pancreatic cancer, large bowel cancer, rectal cancer, colon cancer, prostatic cancer, ovary cancer, uterine cervix cancer, breast cancer, kidney cancer, bladder cancer, brain tumor), a pancreatic disease (for example, acute or chronic pancreatitis, pancreatic cancer), chorioma, hydatidiform mole, invasive mole, miscarriage, fetus hypogenesis, anomaly of saccharometabolism, lipidosis and abnormal childbirth; and

[13] The sustained-release formulation according to item [12] above, wherein the formulation is a microcapsule formulation.

The sustained-release formulation of the present invention slowly and stably releases compound (I) or a salt thereof for a long time and also exerts a medicinal effect of compound (I) or a salt thereof for a long time. Furthermore, the sustained-release formulation of the present invention, which improves patient's convenience by reducing the number of administration times, is an excellent formulation as a clinical medicine.

## DETAILED DESCRIPTION OF THE INVENTION

The present invention will be more specifically described below.

A metastin derivative (in the specification, sometimes simply referred to as compound (I)) to be used in the present invention and represented by the following formula (I) :

Ac-D-Tyr-Hyp-Asn-Thr-Phe-AzaGly-Leu-Arg(Me) -Trp-NH$_2$ (I) (SEQ ID NO:1)

or a salt thereof can be produced by a known peptide synthesis method, more specifically, can be produced by a method described in WO07/72997.

Compound (I) to be used in the present invention may be present in the form of a salt. As the salt formed with compound (I), a pharmacologically acceptable salt is particularly preferable. Examples of such salt include salts with inorganic acids (for example, hydrochloric acid, phosphoric acid, hydrobromic acid, sulfuric acid); salts with organic acids (for example, acetic acid, formic acid, propionic acid, fumaric acid, maleic acid, succinic acid, tartaric acid, citric acid, malic acid, oxalic-acid, benzoic acid, methanesulfonic acid, benzenesulfonic acid); salts with inorganic bases (for example, alkali metal salts such as a sodium salt and potassium salt; alkaline earth metal salts such as a calcium salt and a magnesium salt; an aluminium salt, an ammonium salt) and salts with organic bases (for example, trimethylamine, triethylamine, pyridine, picoline, ethanolamine, diethanolamine, triethanolamine, dicyclohexylamine, N, N-dibenzylethylenediamine).

Preferable examples of the salts formed with compound (I) that can be used in the present invention include salts with acetic acid.

The sustained-release formulation of the present invention contains a tactic acid-glycolic acid copolymer having a weight average molecular weight of 5, 000 to 40, 000 (in the specification, sometimes simply referred to as a lactic acid-glycolic acid copolymer) or a salt thereof

In the present invention, the lactic acid-glycolic acid copolymer or a salt thereof refers to a polymer composed of lactic acid and glycolic acid or a salt thereof The content of glycolic acid in the lactic acid-glycolic acid copolymer to be used in the present invention is beyond 0 wt% and 60 wt% or less, preferably 1 wt% or more and 55 wt% or less, more preferably 5 wt% or more and 50 wt% or less, further preferably 15 wt% or more and 35 wt% or less and particularly preferably about 25 wt%.

The weight average molecular weight of the lactic acid-glycolic acid copolymer to be used in the present invention is 5, 000 to 40, 000, preferably 5, 000 to 30, 000 and further preferably 6, 000 to 20, 000.

The polydispersity of the lactic acid-glycolic acid copolymer (weight average molecular weight /number average molecular weight) is preferably 1.2 to 4.0 and further preferably 1.5 to 3.5.

The weight average molecular weight and the polydispersity used in the specification refer to values obtained by gel permeation chromatographic (GPC) measurement. The weight average molecular weight and the content of each polymer are polystyrene-equivalent weight average molecular weight, which is obtained by GPC measurement using, for example, a mono disperse polystyrene as a standard substance, and the content of each polymer calculated from this, respectively. The weight average molecular weight and the content of each polymer can be measured, for example, by a high-speed GPC apparatus (HLC-8120 GPC manufactured by Tohso Corporation). As a column, Super H4000 x 2 and Super H2000 (each manufactured by Tohso Corporation) can be used. As a mobile phase, tetrahydrofuran can be used and the flow rate can be set to 0.6 mL/min. As a detection method, a differential refractive index can be used

As the lactic acid-glycolic acid copolymer, a commercially available product can he used.

In the present invention, the lactic acid-glycolic acid copolymer may be present in the form of a salt. Examples of the salt include salts with inorganic bases (e.g., alkali metal such as sodium and potassium, alkaline earth metal such as calcium and magnesium) and organic bases (e.g., an organic amine such as triethylamine, a basic amino acid such as arginine) or salts with transition metals (e.g., zinc, iron, copper) and complex salts.

The sustained-release formulation of the present invention is, for example, produced by mixing compound (I) or a salt thereof and a lactic acid-glycolic acid copolymer or a salt thereof, and, if necessary, molding thus-obtained mixture. The amount of compound (I) or a salt thereof to be used is, for example, 0.01 to 50% (w/w) relative to a lactic acid-glycolic acid copolymer or a salt thereof, and preferably 0.1 to 30% (w/w).

Now, the method for producing a sustained-release formulation according to the present invention will be more specifically described below.

**(1) Method for producing rod-form molding and the like**

(1-a) A lactic acid-glycolic acid copolymer or a salt thereof is dissolved in an organic solvent (preferably dichloromethane, etc.) and an aqueous solution of compound (I) or a salt thereof is added and then emulsified. The resulting emulsion is dried in a vacuum to obtain powder having compound (I) or a salt thereof and the lactic acid-glycolic acid copolymer or a salt thereof uniformly dispersed therein. The powder is warmed and cooled to obtain a molding of disk form, film form and rod form, etc. The warming temperature is, for example, 50 to 100°C and cooling temperature is, for example, 0 to 40°C. The amount of compound (I) or a salt thereof to be used varies depending upon the type of compound (I) or a salt thereof, desired pharmacological effect and duration of the effect, etc.; however, it is, for example, 0.41 to 50% (w/w) relative to the lactic acid-glycolic acid copolymer or a salt thereof, preferably 0.1 to 30% (w/w), and particularly preferably 1 to 20% (w/w).

(1-b) A lactic acid-glycolic acid copolymer or a salt thereof is dissolved in an organic solvent (preferably dichloromethane, etc.) and compound (I) or a salt thereof is uniformly dispersed. The resulting dispersion is dried in a vacuum to obtain powder of the lactic acid-glycolic acid copolymer or a salt thereof in which compound (I) or a salt thereof is uniformly dispersed. The powder was warmed and cooled to obtain a molding of disk form, film form and rod form, etc. The warming temperature, cooling temperature and the amount of compound (I) or a salt thereof to be used are the same as described in the above section (1-a).

**(2) Method for producing microcapsules (also referred to as microsphere)**

**(2-a) In-water drying method**

Microcapsules are obtained by subjecting (i) a W (internal water phase) /O (oil phase) /W (external water phase) emulsion, which is obtained by emulsifying a W (internal water phase) /O (oil phase) emulsion composed of an internal water phase containing compound (I) or a salt thereof and an oil phase containing a lactic acid-glycolic acid copolymer or a salt thereof, or (ii) an O (oil phase) /W (external water phase) emulsion, which is obtained by emulsifying an oil phase containing compound (I) or a salt thereof and a lactic acid-glycolic acid copolymer or a salt thereof, to an in-water-drying method.

The emulsion (i), that is, the W/O emulsion, which is composed of an internal water phase containing compound (I) or a salt thereof and an oil phase containing a lactic acid-glycolic acid copolymer or a salt thereof, is produced as follows.

First, compound (I) or a salt thereof is dissolved in water, dispersed or suspended to produce the internal water phase. The concentration of compound (1) or a salt thereof in water is, for example, 0.001 to 90% (w/w) and preferably 0.01 to 80% (w/w).

The amount of compound (I) or a salt thereof to be used varies depending upon the type of compound (I) or a salt thereof, desired pharmacological effect and duration of the effect, etc.; however, it is, for example, 0.01 to 50% (w/w) relative to a lactic acid-glycolic acid copolymer or a salt thereof, preferably 0.1 to 30% (w/w) and further preferably 1 to 20% (w/w).

If necessary, to enhance uptake of compound (I) or a salt thereof in a microcapsule, a drug retaining substance such as gelatin, agar, sodium alginate, polyvinyl alcohol or basic amino acid (for example, arginine, histidine, lysine), may be added to an internal water phase. The addition amount of drug retaining substance is usually 0.01 to 10 fold by weight relative to compound (I) or a salt thereof

Furthermore, if necessary, an amino acid (e.g., tryptophan or arginine) may be added to the internal water phase. Addition of the amino acid may sometimes prevent reduction of blood concentration of compound (I) or a salt thereof in the initial period (up to about 2 weeks after administration) after the sustained-release formulation of the present invention is administered. The addition amount of the amino acid is usually 0.01 to 10 fold by weight relative to compound (I) or a salt thereof preferably 0.05 to 10 fold by weight and further preferably 0.1 to 5 fold by weight.

The internal water phase may be once lyophilized into powder and thereafter dissolved by adding water so as to obtain an appropriate concentration and then put in use.

Separately, a lactic acid-glycolic acid copolymer or a salt thereof is dissolved in an organic solvent to produce an oil phase.

Examples of the organic solvent include halogenated hydrocarbons (for example; dichloromethane, chloroform, chloroethane, trichloroethane, carbon tetrachloride), fatty acid esters (for example, ethyl acetate, butyl acetate) and aromatic hydrocarbons (for example, benzene, toluene, xylene). Of them, dichloromethane is preferable.

The concentration of a lactic acid-glycolic acid copolymer or a salt thereof in an organic solvent varies depending upon the type and weight average molecular weight of lactic acid-glycolic acid copolymer or a salt thereof and the type of organic solvent; however, a value expressed by the expression:

$$[\text{weight of a lactic acid-glycolic acid copolymer or a salt thereof}/(\text{weight of an organic solvent} + \text{weight of a lactic acid-glycolic acid copolymer or a salt thereof})] \, (\times\,100\%)$$

is usually 0.01 to 90% (w/w) and preferably 0.01 to 70% (w/w). The oil phase desirably contains no insoluble matter.

To the organic solvent solution (oil phase) of a lactic acid-glycolic acid copolymer or a salt thereof thus obtained, an aqueous solution, dispersion or suspension (internal water phase) of compound (I) or a salt thereof is added, dispersed and emulsified by a homomixer, etc., to produce a W/O emulsion.

When the W/O emulsion is produced at room temperature (about 19 to 25°C), the resulting W/O emulsion changes with the passage of time to a state (e.g., gelatinous state), which is unfavorable to secondary emulsification (later described). In this case, it is sometimes difficult to produce microcapsules in high yield (the yield used herein refers to a ratio of the weight of compound (I) or a salt thereof contained in microcapsules to the weight of compound (I) or a salt thereof used for a W/O emulsion).

To prevent such a change, it is preferred that a W/O emulsion is produced at a temperature of 31°C or more (preferably 31 to 33°C).

On the other hand, the above emulsion (ii), that is, the oil phase, which contains compound (I) or a salt thereof and a lactic acid-glycolic acid copolymer or a salt thereof, is produced as follows.

First, an organic solvent solution of a lactic acid-glycolic acid copolymer or a salt thereof is produced As the organic solvent, the same organic solvent used for producing the above W/O emulsion is used

The concentration of a lactic acid-glycolic acid copolymer or a salt thereof in an organic solvent solution varies depending upon the type and weight average molecular weight of lactic acid-glycolic acid copolymer or a salt thereof and the type of organic solvent; however, a value expressed by the expression:

$$[\text{weight of a lactic acid-glycolic acid copolymer}/(\text{weight of an organic solvent} + \text{weight of a lactic acid-glycolic acid copolymer}] \, (\times\,100\%)$$

is usually 0.01 to 70% (w/w) and preferably 1 to 60% (w/w).

Next, compound (I) or a salt thereof is dissolved or suspended in the organic solvent solution of lactic acid-glycolic acid copolymer or a salt thereof to prepare an oil phase. The oil phase can be produced also by dissolving or suspending a solution, which is prepared by dissolving compound (I) or a salt thereof in an alcohol, in the organic solvent solution of a lactic acid-glycolic acid copolymer or a salt thereof Examples of the alcohol for dissolving compound (I) or a salt thereof include methanol. As the alcohol, a solution mixture of acetic acid-alcohol (e.g., an acetic acid-methanol mixed solution) containing acetic acid can be used. The content of acetic acid in the acetic acid-alcohol mixed solution is usually 0.1 to 100 fold by weight relative to the weight of alcohol. To the acetic acid-alcohol mixed solution, an amino acid (tryptophane or arginine, etc.) may be added. Use of the amino acid may sometimes prevent reduction of blood concentration of compound (I) or a salt thereof in the initial period (up to about 2 weeks after administration) after the sustained-release formulation of the present invention is administered. The addition amount of amino acid is usually 0.0002 to 0.2 fold by weight relative to the weight of an alcohol, preferably 0.001 to 0.2 fold by weight and further preferably 0.001 to 0.05 fold by weight.

The amount of compound (I) or a salt thereof to be used may be selected such that the ratio of compound (I) or a salt thereof relative to a lactic acid-glycolic acid copolymer or a salt thereof is equal to that employed in producing the above (i) W/O emulsion.

Subsequently, the above (i) W/O emulsion or (ii) oil phase is added to an external water phase, dispersed and emulsified (secondary emulsification) by a homomixer, etc. to produce an emulsion (hereinafter, the emulsion obtained from the W/O emulsion is sometimes referred to as a W/O/W emulsion, whereas the emulsion obtained from the (ii) oil phase is sometimes referred to as an O/W emulsion).

The amount of external water phase to be used is usually 1 to 10, 000 fold by volume relative to the W/O emulsion or oil phase, preferably 10 to 5, 000 fold by volume and particularly preferably 50 to 1, 000 fold by volume.

To the external water phase, an emulsifier is usually added. As the emulsifier, any emulsifier can be used as long as it can usually form a stable W/O/W emulsion or O/W emulsion. Examples thereof include an anionic surfactant, a nonionic surfactant, a polyoxyethylene castor oil derivative, polyvinylpyrrolidone, polyvinyl alcohol, carboxymethylcellulose, lecithin, gelatin and hyaluronic acid. Of them, polyvinyl alcohol is preferable. The concentration of an emulsifier in an external water phase is usually 0.001 to 20% (w/w), preferably 0.01 to 10% (w/w) and particularly preferably 0.05 to 5% (w/w).

The W/O/W emulsion or O/W emulsion (hereinaiter, these each may sometimes simply be referred to as an emulsion) thus obtained is subjected to an in-water-drying method to remove an organic solvent contained in the emulsion. In this manner, microcapsules can be produced.

Furthermore, other than the method using the aforementioned W/O/W emulsion or O/W emulsion, there is a production method in which an S (solid phase) /O (oil phase) emulsion, which is composed of a solid phase containing compound (I) or a salt thereof and an oil phase containing a lactic acid-glycolic acid copolymer or a salt thereof, is subjected to an in-water-drying method.

First, a lactic acid-glycolic acid copolymer or a salt thereof is dissolved in an organic solvent. In the resulting organic solvent solution, compound (I) or a salt thereof is dispersed. At this time, the amounts of compound (I) or a salt thereof and lactic acid-glycolic acid copolymer or a salt thereof to be used may be selected such that the ratio of compound (I) or a salt thereof to the lactic acid-glycolic acid copolymer or a salt thereof becomes the same as in producing the above (i) W/O emulsion. As the organic solvent, the same organic solvent as used in producing the W/O emulsion is used. Furthermore, to uniformly disperse compound (I) or a salt thereof in the organic solvent, for example, ultrasonic irradiation, a turbine-form stirrer or a homogenizer, is used.

Next, the S/O emulsion thus prepared is further added to an external water phase and dispersed and emulsified by use of, for example, ultrasonic irradiation, a turbine-form stirrer or a homogenizer to produce an emulsion (hereinafter sometimes referred to as an S (solid phase) /O (oil phase) /W (water phase) emulsion). Thereafter, the oil-phase solvent is vaporized to produce microcapsules. At this time, the volume of the water phase is generally selected from 1 fold to 10, 000 fold of the oil phase by volume, further preferably 10 fold to 5, 000 fold and particularly preferably 50 fold to 1, 000 fold.

To the external water phase, an emulsifier as mentioned above may be added. The use amount of external water phase and the type and concentration of emulsifier to be added to the external water phase are the same as employed in producing the above W/O/W emulsion.

The S/O/W emulsion thus obtained is subjected to an in-water-drying method to remove an organic solvent. In this manner, microcapsules can be produced.

The microcapsules obtained by using a W/O/W emulsion, an O/W emulsion, an S/O/W emulsion were separated by centrifugation, sieving or filtration, and then, if necessary, washed with distilled water to remove an emulsified, etc. attached to the surface of microcapsules. Thereafter, the microcapsules are dispersed in distilled water, etc., lyophilized, and, if necessary, warmed to further remove water and an organic solvent in the microcapsule. Warming may be performed under reduced pressure. As the conditions for a warming step, heat dry is performed at a temperature, which is not less than a glass-transition temperature of the lactic acid-glycolic acid copolymer used and at which microcapsule particles are not adhered to each other. Preferably, heat dry is performed in the temperature range from the glass-transition temperature of a lactic acid-glycolic acid copolymer or a salt thereof to a temperature higher by about 30°C thereof The glass-transition temperature used herein refers to a medium point of the temperatures obtained by measuring using a differential scanning calorimeter at a temperature raising rate of 10 to 20°C/minute.

**(2-b) Phase separation method**

When microcapsules are produced by this method, a coacervation agent is gradually added under stirring to the W/O emulsion described in the above (2-a) in-water-drying method. In this manner, microcapsules are precipitated and solidified. The amount of the coacervation agent is selected from 0.01 to 1, 000 fold of oil phase volume, preferably 0.05 to 500 fold and particularly preferably 0.1 to 200 fold.

The coacervation agent is not particularly limited as long as it is a polymer compound miscible with an organic solvent, a mineral-oil based compound or a vegetable-oil based compound, etc., and does not dissolve a lactic acid-glycolic acid copolymer or a salt thereof Specific examples thereof that may be used include silicon oil, sesame oil, soybean oil, corn oil, cotton seed oil, coconut oil, linseed oil, mineral oil, n-hexane and n-heptane. These may be used as a mixture of two types or more.

After the microcapsules thus obtained are separated, they are repeatedly washed with heptane, etc. to remove the coacervation agent, etc. and dried under reduced pressure. Alternatively, washing is performed in the same manner as described in the above (2-a) in-water-drying method, followed by lyophilizing and drying by warming.

**(2-c) Spray drying method**

When microcapsules are produced by this method, the W/O emulsion described in the above (2-a) in-water-drying method is sprayed by a nozzle in a dehydration chamber of a spray drier to volatilize the organic solvent within micro liquid drops within an extremely short time. Examples of the nozzle include a two-fluid nozzle type, a pressure nozzle type and a rotation disk type. Thereafter, if necessary, washing is performed in the same manner as described in the above (2-a) in-water-drying method and thereafter may be lyophilized and further dried by warning.

As the dosage form other than the aforementioned microcapsule, microparticles may be mentioned, which are obtained by drying W/O emulsion described in the above (2-a) in-water-drying method, for example, by a rotary evaporator, into a solid, while vaporizing an organic solvent and water by controlling the degree of vacuum, and thereafter pulverizing by a jet mill, etc.

Furthermore, the microparticles pulverized are washed in the same manner as described in the above (2-a) in-water-drying method and thereafter may be lyophilized and further dried by warming.

In dispersing the microcapsules produced in the above sections (2-a), (2-b) or (2-c) in distilled water, etc., an aggregation preventing agent may be added in order to prevent aggregation ofparticles with each other. Examples of the aggregation preventing agent include water soluble polysaccharides such as mannitol, lactose, glucose, a starch (for example, cornstarch) and hyaluronic acid or alkali metal salt thereof; proteins such as glycine, fibrin and collagen; and inorganic salts such as sodium chloride and sodium hydrogenphosphate. Of them, mannitol is preferable. The amount of aggregation preventing agent to be used is preferably 2 to 100 parts by weight relative to microcapsule (100 parts by weight) and further preferably 10 to 25 parts by weight.

Furthermore, microcapsules are warmed and then cooled in the same manner as described in the case of the above (1-a) to obtain a molding of disk form, film form and rod form, etc.

The content of compound (I) or a salt thereof in a microcapsule is not particularly limited; however, with respect to one-month sustained-release formulation, the content thereof is for example, 4 wt% or more to 10 wt% or less and preferably 6 wt% or more to 10 wt% or less.

In various production methods as mentioned above, in dissolving a lactic acid-glycolic acid copolymer or a salt thereof in an organic solvent, zinc oxide may be added to the organic solvent.

The amount of zinc oxide to be used is, for example, 0.01 to 100 parts by weight relative to a tactic acid-glycolic acid copolymer or a salt thereof (100 parts by weight), preferably 0.1 to 20 parts by weight.

Furthermore, the particle size of zinc oxide is usually 0.001 to 10 $\mu$m and preferably 0.005 to 1 $\mu$m.

Likewise, the sustained-release formulation obtained by using zinc oxide has excellent properties, more specifically, "the uptake rate of a drug is high" and "a drug can be persistently released for a long time" etc.

In producing the sustained-release formulation of the present invention, compound (I) or a salt thereof may be dissolved in an aqueous solution of a volatile salt, for example, ammonium acetate, lyophilized and then put in use.

The lyophilized product of compound (I) or a salt thereof obtained by treating with ammonium acetate in this way has a small particles size and excellent operability, and thus advantageous in producing a sustained-release formulation.

To the sustained-release formulation of the present invention thus obtained, if desired, pharmaceutically acceptable additives (for example, a stabilizer, a preservative, a soothing agent) may be appropriately added. Examples of the dosage form of the sustained-release formulation of the present invention include parenteral agents (for example, an injection, an implantation, a suppository) and oral administration agents (for example, a solid formulation such as a capsule agent, a tablet, a granule, a powder; liquid formulation such as a syrup, an emulsion and a suspension). Examples of the stabilizer include human serum albumin and polyethylene glycol. Examples of the preservative include benzyl alcohol and phenol. Examples of the soothing agent include benzalkonium chloride and procaine hydrochloride. In the sustained-release formulation of the present invention, the content of compound (I) or a salt thereof is usually and appropriately selected within the range of 0.01 to 33% (w/w) relative to the total sustained-release formulation.

A one-month sustained-release formulation produced by a method of using a W/O/W emulsion containing compound (I) or a salt thereof can maintain a blood drug concentration at a further higher level during a sustained-release period and thus is more excellent than a one-month sustained-release formulation produced from an O/W emulsion.

The sustained-release formulation of the present invention is excellent in that a blood drug concentration of compound (I) or a salt thereof is stable in a sustained-release period.

The sustained-release formulation of the present invention is preferably a parenteral agent and further preferably an injection. For example, when the sustained-release formulation is in the form of microcapsules, the microcapsules are used in combination with a dispersant (e.g., a surfactant such as Tween 80 and HCO-60; a polysaccharide such as carboxymethylcellulose, sodium alginate and hyaluronic acid), a preservative (e.g., methylparaben, propylparaben) and an isotonic agent (e.g., sodium chloride, mannitol, sorbitol, glucose) etc. to prepare an aqueous suspension. In this manner, a sustained-release injection can be obtained. Furthermore, a sustained-release injection can be obtained also by dispersing microcapsules in a vegetable oil such as sesame oil and corn oil or in the vegetable oil to which a phospholipid such as lecithin is added, or in a medium chain triglyceride (e.g., Miglyol 812) to obtain an oily suspension.

When sustained-release formulation is, for example, in the form of microcapsules, the particle size of microcapsules that are used as a suspension injection may be satisfactory if it satisfies the polydispersity and the range passing through a syringe needle. As an average particles size thereof, for example, the range of 0.1 to 300 μm may be mentioned. The average particle size thereof preferably falls within the range of 1 to 150 μm and particularly preferably 2 to 100 μm.

The aforementioned microcapsules are aseptically treated by a method of performing the whole production steps in aseptic conditions, a method of sterilizing with gamma ray and a method of adding an aseptic agent. The method is not particularly limited.

Since the sustained-release formulation of the present invention is low in its toxicity, it can be stably administered orally or parenterally to mammals (for example, human, monkey, hamadryas, chimpanzee, pig, cow, sheep, horse, dog, cat, mouse, rat).

The sustained-release formulation of the present invention can be used for treating or preventing all diseases in which a physiological activity of metastin is involved. In particular, the sustained-release formulation of the present invention can be effectively used in treating or preventing cancer (for example, lung cancer, stomach cancer, liver cancer, pancreatic cancer, large bowel cancer, rectal cancer, colon cancer, prostatic cancer, ovary cancer, uterine cervix cancer, breast cancer, kidney cancer, bladder cancer, brain tumor), a pancreatic disease (for example, acute or chronic pancreatitis), chorioma, hydatidiform mole, invasive mole, miscarriage, fetus hypogenesis, anomaly of saccharometabolism, lipidosis and abnormal childbirth.

The sustained-release formulation of the present invention is particularly useful as a therapeutic agent or prophylactic agent for cancer (preferably prostatic cancer, more preferably androgen-independent prostatic cancer).

The dose of the sustained-release formulation of the present invention can be appropriately selected depending upon the type and content of compound (I) or a salt thereof serving as an effective ingredient, dosage form, duration of release, administration target, administration route, administration purpose, target disease and symptom, etc.; however, the dose may be satisfactory as long as the effective ingredient can be maintained in a living body in a pharmaceutically effective concentration in a desired duration. For example, in the therapy for an adult cancer patient, when the sustained-release formulation of the present invention is administered, for example, as about one month sustained-release injection, compound (1) or a salt thereof is used in an amount of, for example, within the range of 0.01 to 4 mg/kg body weight, and preferably 0.03 to 0.6 mg/kg body weight per administration. Furthermore, when the sustained-release formulation of the present invention is administered, for example, as an about three-month sustained-release injection, compound (I) or a salt thereof is used in an amount of, for example, within the range of 0.03 to 12 mg/kg body weight, and preferably 0.09 to 1.8 mg/kg body weight per administration. The administration frequency is, for example, once per 3 weeks, once per month, or once per three months, and can be appropriately selected depending upon the content of compound (I) or a salt thereof, dosage form, duration of release, a target disease and administration target, etc. As the sustained-release formulation of the present invention, preferably a 3-week to 4-month sustained-release formulation, and further preferably a 1-month to 4-month sustained-release formulation are mentioned.

Furthermore, the sustained-release formulation of the present invention can be used in combination with other medicines (hereinafter, simply referred to as a combined medicine) for various diseases for which compound (I) or a salt thereof pharmaceutically effectively works, in particular, medicinal agents such as a chemotherapeutic agent, a hormonal therapeutic agent and an immunotherapeutic agent for cancer treatment. At this time, the administration period of the sustained-release formulation of the present invention and the combined medicine is not limited. They can be administered to an administration target simultaneously or at a time interval The dosage amount of a combined medicine can be appropriately selected based on clinical dosage amount. Furthermore, a blending ratio of the sustained-release formulation of the present invention and a combined medicine can be appropriately selected depending upon an administration target, administration route, target disease, symptom and combination, etc.

Examples of the chemotherapeutic agent include alkylating agents (for example, cyclophosphamide, ifosfamide, nimustine, ranimustine, carboquone), antimetabolites (for example, methotrexate, 5-fluorouracil, tegafur, carmofur, UFT, doxifluridine, cytarabine, enocitabine, mercaptopurine, mercaptopurine riboside, thioguanine), anticancer antibiotic substances (for example, mitomycin, adriamycin, daunorubicin, epirubicin, pirarubicin, idarubicin, bleomycin, peplomycin, actinomycin) and plant-derived anticancer agents (for example, vincristine, vinblastine, vindesine, etoposide, camptothecine, irinotecan), cisplatin, carboplatin, nedaplatin, paclitaxel, docetaxel and estramustine.

Examples of the hormonal therapeutic agent include, adrenocortical hormones (for example, prednisolone, prednisone, dexamethasone, cortisone acetate), estrogens (for example, estradiol, ethinylestradiol, fosfestrol, chlorotrianisene), antiestrogen (for example, epitiostanol, mepitiostane, tamoxifen, clomiphene), progesterons (for example, hydroxyprogesterone caproate, dydrogesterone, medroxyprogesterone, norethisterone, norethindrone) and LHRH derivatives (for example, leuprorelin acetate).

Examples of the immunotherapeutic agent include microbial or bacterial components (for example, a muramyldipeptide derivative, picibanil), polysaccharides having an immunological-enhancing activity (for example, lentinan, sizofiran, krestin), cytokines obtained by a genetic engineering approach (for example, interferon, interleukin 2(IL-2), interleukin 12(IL- 12), tumor necrosis factor (TNF)) and colony stimulating factors (for example, granulocyte colony stimulating factor, erythropoietin).

Furthermore, medicines that are confirmed to have an effect of ameliorating cachexia in animal models or clinical practice; more specifically, cyclooxygenase inhibitors (for example, indomethacin) [Cancer Research, Vol. 49, pages 5935 to 5939, 1989], progesterone derivatives (for example, megesterol acetate) [Journal of Clinical Oncology, Vol. 12, pages 213 to 225, 1994], glucocorticosteroids (for example, dexamethasone), metoclopramide based medicines, tetrahydrocannabinol based medicines (literatures are the same as mentioned above), fat metabolism improving agents (for example, eicosapentaenoic acid) [British Journal of Cancer, Vol. 68, pages 314 to 318, 1993], growth hormone, IGF-1, or antibodies against a factor of inducing cachexia, i.e., TNF-$\alpha$, LIF, IL-6, oncostatin M can be used in combination with the sustained-release formulation of the present invention.

Other than these, general medicines for use in treating or preventing diseases of the placenta and pancreas can be used as combined medicines. Examples of such medicines include an anti-inflammatory agent, an antipyretic/analgesic agent, an antibacterial agent, an antiviral agent and a hormonal agent that are clinically used in general.

In the specification, when bases and amino acids, etc. are expressed by abbreviations, they are expressed based on IUPAC IUB Commission on Biochemical Nomenclature or conventional abbreviations routinely used in the art. Examples thereof are as follows. When an optical isomer of an amino acid is conceivably present, unless otherwise specified, an L-form amino acid is shown.

| | |
|---|---|
| Ac | : acetyl |
| AzaGly | : azaglycine |
| Hyp | : trans-4-hydroxyproline |
| Leu | : leucine |
| Thr | : threonine |
| Arg(Me) | : Nw-methyl arginine |
| Phe | : phenyl alanine |
| Tyr | : tyrosine |
| Trp | : tryptophan |
| Asn | : asparagine |

## EXAMPLES

The present invention will be more specifically explained below by way of Examples and Test Examples; however, the present invention is not limited thereto.

In prescriptions described as Examples, as components (additives) other than an active component, substances described, e.g., in the Japanese Pharmacopeia, 15th revision, Japanese Standards for Pharmaceutical Ingredients or adaptive substances listed in the standard for pharmaceutical additive 2003 were used.

## Example 1

A lactic acid-glycolic acid copolymer (lactic acid/glycolic acid = 75/25, weight average molecular weight Mw: 7, 900, number average molecular weight Mn: 3, 400, Mw/Mn ratio: 2.3; manufactured by Wako Pure Chemical Industries Ltd.) (6.2469 g) was dissolved in dichloromethane (10.9328 g). This solution (13.2188 g) was weighed and blended with a solution prepared by dissolving an acetate of compound (I) (1.4796 g) in methanol (5.6505 g) to obtain an oil phase. Subsequently, the oil phase was poured in a 0.1%(w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7, 000 rpm). The O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMACCR 5DL, manufactured by Hitachi, Ltd.). This was dispersed again in distilled water and further centrifuged to wash away a free medicine, etc. The collected microspheres were redispersed in a small amount of distilled water and mannitol (0.8075 g) was added. The mixture was lyophilized by a lyophilizer (DF-01H, ULVAC) to obtain microcapsule powder. The content of compound (I) in the resulting microcapsule powder was 15.5%.

## Example 2

Lactic acid-glycolic acid copolymers (lactic acid/glycolic acid = 75/25, weight average molecular weight Mw: 14, 000, number average molecular weight Mn: 5, 500, Mw/Mn ratio: 2.5; manufactured by Wako Pure Chemical Industries Ltd.) (5.9815 g, 5.9824 g, 5.9846 g, 5.9825 g) were respectively dissolved in dichloromethane (10.9244 g, 11.0231 g, 10.9199 g, 11.0646 g). These solutions (13.4331 g, 13.3842 g, 13.0452 g, 13.0603 g) were separately weighed, blended with aqueous solutions, which were prepared by dissolving an acetate of compound (I) (0.5744 g, 0.5739 g, 0.5752 g, 0.5707 g) respectively in distilled water (0.5333 g, 0.5318 g, 0.5597 g, 0.5479 g) and emulsified by use of a small homogenizer (KINEMATICA) to form W/O emulsions (rotation number: about 10, 000 rpm, 30 sec). Subsequently, the W/O emulsions were respectively poured in four 0.1% (w/w) aqueous solutions (1 liter) of polyvinyl alcohol (EG-40, manufactured by Nippon Synthetic Chemical Industry Co., Ltd.), which were previously adjusted to about 18°C, and subjected to secondary emulsification using a turbine-form homomixer (manufactured by Tokushukika) to prepare W/O/W emulsions (evolution of turbine: about 7, 000 rpm). The W/O/W emulsions each were stirred for about 3 hours (in-water-drying method step), sieved by use of a 75 $\mu$m standard sieve, and then microspheres were collected by centrifugation (rotation number: about 2, 500 rpm, 5 min) using a centrifuge (HIMACCR 5DL, manufactured by Hitachi, Ltd.). This was dispersed again in distilled water and further centrifuged to wash away a free medicine, etc. The collected microspheres were all redispersed in a small amount of distilled water to put together into a whole and mannitol (2.592 g) was added. The mixture was lyophilized by a lyophilizer (DF-01H, ULVAC) to obtain microcapsule powder. The content of compound (I) in the resulting microcapsule powder was 6.4%.

## Example 3

Lactic acid-glycolic acid copolymers (lactic acid/glycolic acid = 75/25, weight average molecular weight Mw: 7, 900, number average molecular weight Mn: 3, 400, Mw/Mn ratio: 2.3; manufactured by Wako Pure Chemical Industries Ltd.) (5.9852 g, 5.9858 g, 5.9857 g, 5.9819 g) were respectively dissolved in dichloromethane (11.0099 g, 10.9211 g, 10.9389 g, 10.9393 g). These solutions (13.0645 g, 13.0548 g, 13.0728 g, 13.0405 g) were separately weighed, blended with aqueous solutions, which were prepared by dissolving an acetate of compound (I) (0.5705 g, 0.5735 g, 0.5736 g, 0.5702 g) respectively in distilled water (0.5552 g, 0.6459 g, 0.5054 g, 0.5292 g) and emulsified by use of a small homogenizer (KINEMATICA) to form W/O emulsions (rotation number: about 10, 000 rpm, 30 sec). Subsequently, the W/O emulsions were respectively poured in four 0.1% (w/w) aqueous polyvinyl alcohol (EG-40, manufactured by Nippon Synthetic Chemical Industry Co., Ltd.) solutions (1 liter), which were previously adjusted to about 18°C, and subjected to secondary emulsification using a turbine-form homomixer (manufactured by Tokushukika) to prepare W/O/W emulsions (evolution of turbine: about 7, 000 rpm). The W/O/W emulsions each were stirred for about 3 hours (in-water-drying method step), sieved by use of a 75 $\mu$m standard sieve, and then, microspheres were collected by centrifugation (rotation number: about 2, 500 rpm, 5 min) using a centrifuge (HIMACCR 5DL, manufactured by Hitachi, Ltd.). This was dispersed again in distilled water and further centrifuged to wash away a free medicine, etc. The collected microsphereswere all redispersed in a small amount of distilled water to put together into a whole and mannitol (2.596 g) was added The mixture was lyophilized by a lyophilizer (DF-01H, ULVAC) to obtain microcapsule powder. The content of compound (I) in the resulting microcapsule powder was 7.7%.

## Example 4

A lactic acid-glycolic acid copolymer (lactic acid/glycolic acid = 65/35, weight average molecular weight Mw: 10, 000, number average molecular weight Mn: 4, 200, Mw/Mn ratio: 2.4; manufactured by Wako Pure Chemical Industries Ltd.) (5.85 g) was dissolved in dichloromethane (10.92 g). This solution (12.9 g) was weighed, blended with an aqueous solution prepared by dissolving an acetate of compound (I) (0.573 g) in distilled water (0.5 g) and emulsified by use of a small homogenizer (KINEMATICA) to form a W/O emulsion (rotation number: about 10, 000 rpm, 30 sec). Subsequently, the W/O emulsion was poured in a 0.1%(w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to obtain a W/O/W emulsion (evolution of turbine: about 7, 000 rpm). The W/O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMACCR 5DL, manufactured by Hitachi, Ltd.). This was dispersed again in distilled water and further centrifuged to wash away a free medicine, etc. The collected microspheres were redispersed in a small amount of distilled water and mannitol (0.85 g) was added. The mixture was lyophilized by a lyophilizer (DF-01H, ULVAC) to obtain microcapsule powder. The content of compound (I) in the resulting microcapsule powder was 6.0%.

**Example 5**

A lactic acid-glycolic acid copolymer (lactic acid/glycolic acid = 65/35, weight average molecular weight Mw: 14, 200, number average molecular weight Mn: 5, 700, Mw/Mn ratio: 2.5; manufactured by Wako Pure Chemical Industries Ltd.) (5.85 g) was dissolved in dichloromethane (10.92 g). This solution (12.9 g) was weighed, and blended with an aqueous solution prepared by dissolving an acetate of compound (I) (0.573 g) in distilled water (0.5 g) and emulsified by use of a small homogenizer (KINEMATICA) to form a W/O emulsion (rotation number: about 10, 000 rpm, 30 sec). Subsequently, the W/O emulsion was poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMACCR 5DL, manufactured by Hitachi, Ltd.). This was dispersed again in distilled water and further centrifuged to wash away a free medicine, etc. The collected microspheres were redispersed in a small amount of distilled water and mannitol (0.85 g) was added. The mixture was lyophilized by a lyophilizer (DF-01H, ULVAC) to obtain microcapsule powder. The content of compound (I) in the resulting microcapsule powder was 6.6%.

**Example 6**

A lactic acid-glycolic acid copolymer (lactic acid/glycolic acid = 50/50, weight average molecular weight Mw: 13, 900, number average molecular weight Mn: 5, 000, Mw/Mn ratio: 2.8; manufactured by Wako Pure Chemical Industries Ltd.) (5.85 g) was dissolved in dichloromethane (10.92 g). This solution (12.9 g) was weighed, blended with an aqueous solution prepared by dissolving an acetate of compound (I) (0.573 g) in distilled water (0.5 g) and emulsified by use of a small homogenizer (KINEMATICA) to form a W/O emulsion (rotation number: about 10, 000 rpm, 30 sec). Subsequently, the W/O emulsion was poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMACCR 5DL, manufactured by Hitachi, Ltd.). This was dispersed again in distilled water and further centrifuged to wash away a free medicine, etc. The collected microspheres were redispersed in a small amount of distilled water and mannitol (0.85 g) was added. The mixture was lyophilized by a lyophilizer (DF-01H, ULVAC) to obtain microcapsule powder. The content of compound (1) in the resulting microcapsule powder was 7.0%.

**Example 7**

A lactic acid-glycolic acid copolymer (lactic acid/glycolic acid = 50/50, weight average molecular weight Mw: 15, 900, number average molecular weight Mn: 5, 400, Mw/Mn ratio: 2.9; manufactured by Wako Pure Chemical Industries Ltd.) (5.85 g) was dissolved in dichloromethane (10.92 g). This solution (12.9 g) was weighed, blended with an aqueous solution prepared by dissolving an acetate of compound (I) (0.573 g) in distilled water (0.5 g) and emulsified by use of a small homogenizer (KINEMATICA) to form a W/O emulsion (rotation number: about 10, 000 rpm, 30 sec). Subsequently, the W/O emulsion was poured in a 0.1% (w/w) aqueous polyvinyl alcohol (EG-40, manufactured by Nippon Synthetic Chemical Industry Co., Ltd) solution (1 liter), which was previously adjusted to about 18°C, and subjected to secondary emulsification using a turbine-fonm homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMACCR 5DL, manufactured by Hitachi, Ltd.). This was dispersed again in distilled water and further centrifuged to wash away a free medicine, etc. The collected microspheres were redispersed in a small amount of distilled water and mannitol (0.85 g) was added. The mixture was lyophilized by a lyophilizer (DF-01H, ULVAC) to obtain microcapsule powder. The content of compound (I) in the resulting microcapsule powder was 6.6%.

## Example 8

A lactic acid-glycolic acid copolymer (lactic acid/glycolic acid = 50/50, weight average molecular weight Mw: 17, 800, number average molecular weight Mn: 4, 900, Mw/Mn ratio: 3.6; manufactured by Wako Pure Chemical Industries Ltd.) (5.85 g) was dissolved in dichloromethane (10.92 g). This solution (12.9 g) was weighed, blended with an aqueous solution prepared by dissolving an acetate of compound (I) (0.573 g) in distilled water (0.5 g) and emulsified by use of a small homogenizer (KINEMATICA) to form a W/O emulsion (rotation number: about 10, 000 rpm, 30 sec). Subsequently, the W/O emulsion was poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-fonm homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 μm standard sieve, and then, microspheres were collected by centrifugation (rotation number: about 2, 500 rpm, 5 min) by use of a centrifuge (HIMACCR 5DL, manufactured by Hitachi, Ltd.). This was dispersed again in distilled water and further centrifuged to wash away a free medicine, etc. The collected microspheres were redispersed in a small amount of distilled water and mannitol (0.85 g) was added. The mixture was lyophilized by a lyophilizer (DF-01H, ULVAC) to obtain microcapsule powder. The content of compound (I) in the resulting microcapsule powder was 6.1%.

## Example 9

A lactic acid-glycolic acid copolymer (lactic acid/glycolic acid = 75/25, weight average molecular weight Mw: 8, 000, number average molecular weight Mn: 3, 500, Mw/Mn ratio: 2.3; manufactured by Wako Pure Chemical Industries Ltd) (42.11 g) was dissolved in dichloromethane (78.81 g). This solution (15.50 g) was weighed, blended with an aqueous solution prepared by dissolving an acetate of compound (I) (0.710 g) in distilled water of (0.60 g) and emulsified by use of a small homogenizer (KINEMATICA) to form a W/O emulsion (rotation number: about 10, 000 rpm, 30 sec). Subsequently, the W/O emulsion was poured in a 0.001%(w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMACCR 5DL, manufactured by Hitachi, Ltd). This was dispersed again in distilled water and further centrifuged to wash away a free medicine, etc. The collected microspheres were redispersed in a small amount of distilled water and mannitol (0.706 g) was added. The mixture was lyophilized by a lyophilizer (DF-01H, ULVAC) to obtain microcapsule powder. The content of compound (I) in the resulting microcapsule powder was 6.7%.

## Example 10

A lactic acid-glycolic acid copolymer (lactic acid/glycolic acid = 75/25, weight average molecular weight Mw: 7, 800, number average molecular weight Mn: 3, 400, Mw/Mn ratio: 2.3; manufactured by Wako Pure Chemical Industries Ltd.) (409.80 g) was dissolved in dichloromethane (757.76 g). This solution (795.45 g) was weighed, blended with an aqueous solution prepared by dissolving an acetate of compound (I) (35.10 g) in distilled water of (30.60 g) and emulsified by use of Robomix (manufactured by Tokushukika) to form a W/O emulsion (rotation number: about 10, 000 rpm, 1 min). Subsequently, the W/O emulsion about was cooled to about 10°C, and then poured in a 0.1%(w/w) aqueous polyvinyl alcohol (EG-40, manufactured by Nippon Synthetic Chemical Industry Co., Ltd.) solution (50 liter), which was previously adjusted to about 18°C, and subjected to secondary emulsification using HOMOMIC LINE FLOW (manufactured by Tokushukika) to obtain a W/O/W emulsion (evolution of turbine: about 7, 000 rpm, circulation pump rotation number: about 2000 rpm). The W/O/W emulsion was stirred for about 3 hours (in-water-drying method step) and sieved by use of a 75 μm standard sieve. Subsequently, microspheres were continuously collected by centrifugation (rotation number: about 2, 000 rpm, flow rate: about 550 ml/min) using a centrifuge (H-600S, manufactured by KOKUSAN Co., Ltd). The collected microspheres were redispersed in a small amount of distilled water and sieved by use of a 90 μm standard sieve and then, mannitol (42.436 g) was added. The mixture was lyophilized by use of a lyophilizer (DFM-05A-S, ULVAC) to obtain microcapsule powder. The content of compound (I) in the resulting microcapsule powder was 8.2%.

Example 11

A lactic acid-glycolic acid copolymer (lactic acid/glycolic acid = 75/25, weight average molecular weight Mw: 8, 200, number average molecular weight Mn: 3, 400, Mw/Mn ratio: 2.4; manufactured by Wako Pure Chemical Industries Ltd.) (1423.4 g) was dissolved in dichloromethane (2626.0 g). This solution (3116 g) was weighed, blended with an aqueous solution prepared by dissolving an acetate of compound (I) (136.12 g) in distilled water (120.02 g), and emulsified by a mini-mixer (manufactured by Tokushukika) to form a W/O emulsion (rotation number: about 5, 800 rpm, 12 min). Subsequently, the W/O emulsion was poured in a 0.1%(w/w) aqueous polyvinyl alcohol (EG-40, manufactured by Nippon Synthetic Chemical Industry Co., Ltd.) solution (200 liter), which was previously adjusted to about 18°C, and subjected to secondary emulsification using HOMOMIC LINE FLOW (manufactured by Tokushukika) to obtain a W/O/W emulsion (evolution of turbine: about 7, 000 rpm, circulation pump rotation number: about 2, 500 rpm). The W/O/W emulsion was stirred for about 3 hours (in-water-drying method step) and sieved by use of a 75 μm standard sieve, and then, microspheres were continuously collected by centrifugation (rotation number: about 2, 000 rpm, flow rate: about 600 ml/min) using a centrifuge (H-1002 special type, manufactured by KOKUSAN Co., Ltd.). This was dispersed again in distilled water and further centrifuged to wash away a free medicine, etc. The collected microspheres were redispersed in a small amount of distilled water and sieved by use of a 90 μm standard sieve, and then, mannitol (169.40 g) was added. The mixture was lyophilized by a lyophilizer (RL-402BS, manufactured by Kyowa Vacuum Engineering. Co., Ltd.) to obtain microcapsule powder. The content of compound (I) in the resulting microcapsule powder was 7.6% and the yield of compound (I) was 63.2%.

**Example 11-2**

A lactic acid-glycolic acid copolymer (lactic acid/glycolic acid = 75/25, weight average molecular weight Mw: 8, 000, number average molecular weight Mn: 3, 239, Mw/Mn ratio: 2.5; manufactured by Wako Pure Chemical Industries Ltd.) (1431.8 g) was dissolved in dichloromethane (2626.0 g). This solution (3121 g) was weighed, blended with an aqueous solution prepared by dissolving an acetate of compound (I) (137.1 g) in distilled water (120.0 g) and emulsified by use of a mini-mixer (manufactured by Tokushukika) to form a W/O emulsion

(rotation number: about 5, 800 rpm, 12 min). Subsequently, the W/O emulsion, which was adjusted to 32°C, was poured in a 0.1% (w/w) aqueous polyvinyl alcohol (EG-40, manufactured by Nippon Synthetic Chemical Industry Co., Ltd.) solution (200 liter), which was previously adjusted to about 18°C, and subjected to secondary emulsification using a turbine-form homomixer (HOMOMIC LINE FLOW: manufactured by Tokushukika) to obtain a W/O/W emulsion (evolution of turbine: about 7, 000 rpm, circulation pump rotation number: about 2, 500 rpm). The W/O/W emulsion was stirred for about 3 hours (in-water-drying method step) and sieved by use of a 75 μm standard sieve, and then, microspheres were continuously collected by centrifugation (rotation number: about 2, 000 rpm, flow rate: about 600 ml/min) using a centrifuge (H-1002 special type, manufactured by KOKUSAN Co., Ltd). This was dispersed again in distilled water and further centrifuged to wash away a free medicine, etc. The collected microspheres were redispersed in a small amount of distilled water and sieved by use of a 90 μm standard sieve, and then, a 15% aqueous mannitol solution (1300 g) was added. The mixture was lyophilized by a lyophilizer (RL-402BS, manufactured by Kyowa Vacuum Engineering. Co., Ltd.) to obtain microcapsule powder. The content of compound (I) in the resulting microcapsule powder was 8.0% and the yield of compound (I) was 81.2%.

It was demonstrated that a microcapsule can be produced in a high yield by adjusting the temperature of the W/O emulsion to 31°C or more (more specifically, 32°C).

**Example 12**

A lactic acid-glycolic acid copolymer (lactic acid/glycolic acid = 75/25, weight average molecular weight Mw: 16, 000, number average molecular weight Mn: 5, 900, Mw/Mn ratio: 2.7; manufactured by Wako Pure Chemical Industries Ltd) (6.2400 g) was dissolved in dichloromethane (10.9393 g). This solution (13.2986 g) was weighed and blended with a solution prepared by dissolving an acetate of compound (I) (1.4467 g) in methanol (5.6115 g) to obtain an oil phase. Subsequently, the oil phase was cooled to about 10°C, and then, poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to obtain an O/W emulsion (evolution of turbine: about 7, 000 rpm). The O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMACCR 5DL, manufactured by Hitachi, Ltd.). This was dispersed again in distilled water and further centrifuged to wash away a free medicine, etc. The collected microspheres were redispersed in a small amount of distilled water and mannitol (0.7430 g) was added. The mixture was lyophilized by a lyophilizer (DF-01H, ULVAC) to obtain microcapsule powder. The content of compound (I) in the resulting microcapsule powder was 14.4%.

## Example 13

A lactic acid-glycolic acid copolymer (lactic acid/glycolic acid = 75/25, weight average molecular weight Mw: 17, 600, number average molecular weight Mn: 6, 100, Mw/Mn ratio: 2.9; manufactured by Wako Pure Chemical Industries Ltd.) (6.2402 g) was dissolved in dichloromethane (10.9499 g). This solution (13.2229 g) was weighed and blended with a solution prepared by dissolving an acetate of compound (I) (1.4367 g) in methanol (5.6085 g) to obtain an oil phase. Subsequently, the oil phase was cooled to about 10°C, and then, poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to obtain an O/W emulsion (evolution of turbine: about 7, 000 rpm). The O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMACCR 5DL, manufactured by Hitachi, Ltd.). This was dispersed again in distilled water and further centrifuged to wash away a free medicine, etc. The collected microspheres were redispersed in a small amount of distilled water and mannitol (0.7425 g) was added. The mixture was lyophilized by a lyophilizer (DF-01H, ULVAC) to obtain microcapsule powder. The content of compound (I) in the resulting microcapsule powder was 13.5%.

## Example 14

A lactic acid-glycolic acid copolymer (lactic acid/glycolic acid = 95/5, weight average molecular weight Mw: 8, 100, number average molecular weight Mn: 3, 500, Mw/Mn ratio: 2.3; manufactured by Wako Pure Chemical Industries Ltd.) (7.03 g) was dissolved in dichloromethane (13.14 g). This solution (15.48 g) was weighed and blended with a solution prepared by dissolving an acetate of compound (I) (0.68 g) in methanol (2.80 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to obtain an O/W emulsion (evolution of turbine: about 7, 000 rpm). The O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMACCR 5DL, manufactured by Hitachi, Ltd.). This was dispersed again in distilled water and further centrifuged to wash away a free medicine, etc. The collected microspheres were redispersed in a small amount of distilled water and mannitol (0.847 g) was added. The mixture was lyophilized by a lyophilizer (DF-01H, ULVAC) to obtain microcapsule powder. The content of compound (I) in the resulting microcapsule powder was 8.1%.

## Example 15

A lactic acid-glycolic acid copolymer (lactic acid/glycolic acid = 85/15, weight average molecular weight Mw: 12, 100, number average molecular weight Mn: 5, 200, Mw/Mn ratio: 2.3; manufactured by Wako Pure Chemical Industries Ltd.) (7.02 g) was dissolved in dichloromethane (13.16 g). This solution (15.59 g) was weighed and blended with a solution prepared by dissolving an acetate of compound (I) (0.68 g) in methanol (2.80 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to obtain an O/W emulsion (evolution of turbine: about 7, 000 rpm). The O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMACCR 5DL, manufactured by Hitachi, Ltd.). This was dispersed again in distilled water and further centrifuged to wash away a free medicine, etc. The collected microspheres were redispersed in a small amount of distilled water and mannitol (0.847 g) was added. The mixture was lyophilized by a lyophilizer (DF-01H, ULVAC) to obtain microcapsule powder. The content of compound (I) in the resulting microcapsule powder was 7.6%.

## Example 16

A lactic acid-glycolic acid copolymer (lactic acid/glycolic acid = 85/15, weight average molecular weight Mw: 14, 300, number average molecular weight Mn: 6, 200, Mw/Mn ratio: 2.3; manufactured by Wako Pure Chemical Industries Ltd.) (6.24 g) was dissolved in dichloromethane (10.92 g). This solution (13.19 g) was weighed and blended with a solution prepared by dissolving an acetate of compound (I) (1.36 g) in methanol (5.59 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to obtain an O/W emulsion (evolution of turbine: about 7, 000 rpm). The O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMACCR 5DL, manufactured by Hitachi, Ltd.). This was dispersed again in distilled water and further centrifuged to wash away a free medicine, etc. The collected microspheres were redispersed in a small amount of distilled water and mannitol (0.855 g) was added. The mixture was lyophilized by a lyophilizer (DF-01H, ULVAC) to obtain microcapsule powder. The content of compound (I) in the resulting microcapsule powder was 14.1%.

## Example 17

A lactic acid-glycolic acid copolymer (lactic acid/glycolic acid = 85/15, weight average molecular weight Mw: 10, 200, number average molecular weight Mn: 4, 400, Mw/Mn ratio: 2.3; manufactured by Wako Pure Chemical Industries Ltd.) (7.02302 g) was dissolved in dichloromethane (13.07 g). This solution (15.25 g) was weighed, blended with an aqueous solution prepared by dissolving an acetate of compound (I) (0.68223 g) in distilled water (0.60332 g) and emulsified by use of a small homogenizer (KINEMATICA) to form a W/O emulsion (rotation number: about 10, 000 rpm, 30 sec). Subsequently, the W/O emulsion was poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMACCR 5DL, manufactured by Hitachi, Ltd.). This was dispersed again in distilled water and further centrifuged to wash away a free medicine, etc. The collected microspheres were redispersed in a small amount of distilled water and mannitol (0.917 g) was added. The mixture was lyophilized by a lyophilizer (DF-01H, ULVAC) to obtain microcapsule powder. The content of compound (I) in the resulting microcapsule powder was 7.4%.

## Example 18

A lactic acid-glycolic acid copolymer (lactic acid/glycolic acid = 85/15, weight average molecular weight Mw: 14, 300, number average molecular weight Mn: 6, 200, Mw/Mn ratio: 2.3; manufactured by Wako Pure Chemical Industries Ltd.) (7.03252 g) was dissolved in dichloromethane (13.14 g). This solution (15.51 g) was weighed, blended with an aqueous solution prepared by dissolving an acetate of compound (I) (0.68946 g) in distilled water (0.59202 g) and emulsified by use of a small homogenizer (KINEMATICA) to form a W/O emulsion (rotation number: about 10, 000 rpm, 30 sec). Subsequently, the W/O emulsion was poured in a 0.1% (w/w) aqueous polyvinyl alcohol (EG-40, manufactured by Nippon Synthetic Chemical Industry Co., Ltd) solution (1 liter), which was previously adjusted to about 18°C, and subjected to secondary emulsification using a turbine-form homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMACCR 5DL, manufactured by Hitachi, Ltd.). This was dispersed again in distilled water and further centrifuged to wash away a free medicine, etc. The collected microspheres were redispersed in a small amount of distilled water and mannitol (0.838 g) was added. The mixture was lyophilized by a lyophilizer (DF-01H, ULVAC) to obtain microcapsule powder. The content of compound (I) in the resulting microcapsule powder was 7.3%.

## Example 19

A lactic acid-glycolic acid copolymer (lactic acid/glycolic acid = 85/15, weight average molecular weight Mw: 12, 100, number average molecular weight Mn: 5, 200, Mw/Mn ratio: 2.3; manufactured by Wako Pure Chemical Industries Ltd.) (6.25043 g) was dissolved in dichloromethane (10.95 g). This solution (13.25 g) was weighed, blended with an aqueous solution prepared by dissolving an acetate of compound (I) (1.36512 g) in distilled water (1.20535 g) and emulsified by use of a small homogenizer (KINEMATICA) to form a W/O emulsion (rotation number: about 10, 000 rpm, 30 sec). Subsequently, the W/O emulsion was poured in a 0.1% (w/w) aqueous polyvinyl alcohol (EG-40, manufactured by Nippon Synthetic Chemical Industry Co., Ltd) solution (1 liter), which was previously adjusted to about 18°C, and subjected to secondary emulsification using a turbine-form homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMACCR 5DL, manufactured by Hitachi, Ltd). This was dispersed again in distilled water and further centrifuged to wash away a free medicine, etc. The collected microspheres were redispersed in a small amount of distilled water and mannitol (0.852 g) was added. The mixture was lyophilized by a lyophilizer (DF-01H, ULVAC) to obtain microcapsule powder. The content of compound (I) in the resulting microcapsule powder was 12.4%.

## Example 20

A lactic acid-glycolic acid copolymer (lactic acid/glycolic acid = 85/15, weight average molecular weight Mw: 14, 300, number average molecular weight Mn: 6, 200, Mw/Mn ratio: 2.3; manufactured by Wako Pure Chemical Industries Ltd.) (6.24105 g) was dissolved in dichloromethane (10.93 g). This solution (13.21 g) was weighed, blended with an aqueous  solution prepared by dissolving an acetate of compound (I) (1.36150 g) in distilled water (1.20335 g) and emulsified by use of a small homogenizer (KINEMATICA) to form a W/O emulsion (rotation number: about 10, 000 rpm, 30 sec). Subsequently, the W/O emulsion was poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMACCR 5DL, manufactured by Hitachi, Ltd). This was dispersed again in distilled water and further centrifuged to wash away a free medicine, etc. The collected microspheres were redispersed in a small amount of distilled water and mannitol (0.855 g) was added. The mixture was lyophilized by a lyophilizer (DF-01H, ULVAC) to obtain microcapsule powder. The content of compound (I) in the resulting microcapsule powder was 11.8%.

## Example 21

A lactic acid-glycolic acid copolymer (lactic acid/glycolic acid = 95/5, weight average molecular weight Mw: 10, 100, number average molecular weight Mn: 4, 100, Mw/Mn ratio: 2.5; manufactured by Wako Pure Chemical Industries Ltd.) (7.0461 g) was dissolved in dichloromethane (13.33 g). This solution (15.55 g) was weighed and blended with a solution prepared by dissolving an acetate of compound (I) (0.6874 g) in methanol (2.84 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to obtain an O/W emulsion (evolution of turbine: about 7, 000 rpm). The O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMAC CR 5DL, manufactured by Hitachi, Ltd). This was dispersed again in distilled water and further centrifuged to wash away a free medicine, etc. The collected microspheres were redispersed in a small amount of distilled water and mannitol (0.853 g) was added. The mixture was lyophilized by a lyophilizer (DF-01H, ULVAC) to obtain microcapsule powder. The content of compound (I) in the resulting microcapsule powder was 8.0%.

## Example 22

A lactic acid-glycolic acid copolymer (lactic acid/glycolic acid = 95/5, weight average  molecular weight Mw: 12, 200, number average molecular weight Mn: 4, 900, Mw/Mn ratio: 2.5; manufactured by Wako Pure Chemical Industries Ltd) (7.0265 g) was dissolved in dichloromethane (13.21 g). This solution (15.52 g) was weighed and blended with a solution prepared by dissolving an acetate of compound (I) (0.6837 g) in methanol (2.89 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to obtain an O/W emulsion (evolution of turbine: about 7, 000 rpm). The O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.857 g) was added. The mixture was lyophilized by a lyophilizer (DF-01H, ULVAC) to obtain microcapsule powder. The content of compound (I) in the resulting microcapsule powder was 7.7%.

## Example 23

A lactic acid-glycolic acid copolymer (lactic acid/glycolic acid = 95/5, weight average molecular weight Mw: 14, 000, number average molecular weight Mn: 5, 900, Mw/Mn ratio: 2.4; manufactured by Wako Pure Chemical Industries Ltd.) (13.25758 g) was dissolved in dichloromethane (24.09 g). This solution (15.57 g) was weighed and blended with a solution prepared by dissolving an acetate of compound (I) (0.7413 g) in methanol (2.81 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to obtain an O/W emulsion (evolution of turbine: about 7, 000 rpm). The O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.840 g) was added. The mixture was lyophilized by a lyophilizer (DF-01H, ULVAC) to obtain microcapsule powder. The content of compound (I) in the resulting microcapsule powder was 8.3%.

**Example 24**

A lactic acid-glycolic acid copolymer (lactic acid/glycolic acid = 95/5, weight average molecular weight Mw: 16, 300, number average molecular weight Mn: 6, 500, Mw/Mn ratio: 2.5; manufactured by Wako Pure Chemical Industries Ltd.) (13.25830 g) was dissolved in dichloromethane (24.07 g). This solution (15.52 g) was weighed and blended with a solution prepared by dissolving an acetate of compound (I) (0.7396 g) in methanol (2.81 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to obtain an O/W emulsion (evolution of turbine: about 7, 000 rpm). The O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMAC CR 5DL, manufactured by Hitachi, Ltd). This was dispersed again in distilled water and further centrifuged to wash away a free medicine, etc. The collected microspheres were redispersed in a small amount of distilled water and mannitol (0.875 g) was added. The mixture was lyophilized by a lyophilizer (DF-01H, ULVAC) to obtain microcapsule powder. The content of compound (I) in the resulting microcapsule powder was 8.4%.

**Example 25**

A lactic acid-glycolic acid copolymer (lactic acid/glycolic acid = 95/5, weight average molecular weight Mw: 18, 300, number average molecular weight Mn: 6, 900, Mw/Mn ratio: 2.7; manufactured by Wako Pure Chemical Industries Ltd.) (13.26369 g) was dissolved in dichloromethane (24.08 g). This solution (15.51 g) was weighed and blended with a solution prepared by dissolving an acetate of compound (1) (0.7407 g) in methanol (2.81 g) to obtain an oil phase. Subsequently, the oil phase was poured in a 0.1% (w/w) aqueous polyvinyl alcohol (EG-40, manufactured by Nippon Synthetic Chemical Industry Co., Ltd) solution (1 liter), which was previously adjusted to about 18°C, and emulsified by use of a turbine-form homomixer (manufactured by Tokushukika) to obtain an O/W emulsion (evolution of turbine: about 7, 000 rpm). The O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.851 g) was added. The mixture was lyophilized by a lyophilizer (DF-01H, ULVAC) to obtain microcapsule powder. The content of compound (I) in the resulting microcapsule powder was 8.5%.

**Example 26**

A lactic acid-glycolic acid copolymer (lactic acid/glycolic acid = 95/5, weight average molecular weight Mw: 12, 200, number average molecular weight Mn: 4, 900, Mw/Mn ratio: 2.5; manufactured by Wako Pure Chemical Industries Ltd.) (13.26412 g) was dissolved in dichloromethane (24.13 g). This solution (15.54 g) was weighed, blended with an aqueous solution prepared by dissolving an acetate of compound (I) (0.74057 g) in distilled water (0.60272 g) and emulsified by use of a small homogenizer (KINEMATICA) to form a W/O emulsion (rotation number: about 10, 000 rpm, 30 sec). Subsequently, the W/O emulsion, which was adjusted to 32°C, was poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.899 g) was added.

The mixture was lyophilized by a lyophilizer (DF-01H, ULVAC) to obtain microcapsule powder. The content of compound (I) in the resulting microcapsule powder was 6.1%.

**Example 27**

A lactic acid-glycolic acid copolymer (lactic acid/glycolic acid = 95/5, weight average molecular weight Mw: 14, 000, number average molecular weight Mn: 5, 900, Mw/Mn ratio: 2.4; manufactured by Wako Pure Chemical Industries Ltd.) (13.28203 g) was dissolved in dichloromethane (24.08 g). This solution (15.49 g) was weighed, blended with an aqueous solution prepared by dissolving an acetate of compound (I) (0.73955 g) in distilled water (0.60079 g) and emulsified by use of a small homogenizer (KINEMATICA) to form a W/O emulsion (rotation number: about 10, 000 rpm, 30 sec). Subsequently, the W/O emulsion, which was adjusted to 32°C, was poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.896 g) was added. The mixture was lyophilized by a lyophilizer (DF-01H, ULVAC) to obtain microcapsule powder. The content of compound (I) in the resulting microcapsule powder was 6.7%.

**Example 28**

A lactic acid-glycolic acid copolymer (lactic acid/glycolic acid = 95/5, weight average molecular weight Mw: 16, 300, number average molecular weight Mn: 6, 500, Mw/Mn ratio: 2.5; manufactured by Wako Pure Chemical Industries Ltd.) (13.25986 g) was dissolved in dichloromethane (24.05 g). This solution (15.57 g) was weighed, blended with an aqueous solution prepared by dissolving an acetate of compound (I) (0.74347 g) in distilled water(0.60239 g), and emulsified by use of a small homogenizer (KINEMATICA) to form a W/O emulsion (rotation number: about 10, 000 rpm, 30 sec). Subsequently, the W/O emulsion, which was adjusted to 32°C, was poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.857 g) was added. The mixture was lyophilized by a lyophilizer (DF-01H, ULVAC) to obtain microcapsule powder. The content of compound (I) in the resulting microcapsule powder was 6.0%.

**Example 29**

A lactic acid-glycolic acid copolymer (lactic acid/glycolic acid = 95/5, weight average molecular weight Mw: 18, 300, number average molecular weight Mn: 6, 900, Mw/Mn ratio: 2.7; manufactured by Wako Pure Chemical Industries Ltd.) (13.27697 g) was dissolved in dichloromethane (24.03 g). This solution (15.71 g) was weighed, blended with an aqueous solution prepared by dissolving an acetate of compound (I) (0.74460 g) in distilled water (0.60206 g) and emulsified by use of a small homogenizer (KINEMATICA) to form a W/O emulsion (rotation number: about 10, 000 rpm, 30 sec). Subsequently, the W/O emulsion, which was adjusted to 32°C, was poured in a 0.1% (w/w) aqueous polyvinyl alcohol (EG-40, manufactured by Nippon Synthetic Chemical Industry Co., Ltd) solution (1 liter), which was previously adjusted to about 18°C, and subjected to secondary emulsification using a turbine-form homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.851 g) was added. The mixture was lyophilized by a lyophilizer (DF-01H, ULVAC) to obtain microcapsule powder. The content of compound (I) in the resulting microcapsule powder was 5.3%.

### Example 30

A lactic acid-glycolic acid copolymer (lactic acid/glycolic acid = 95/5, weight average molecular weight Mw: 8, 100, number average molecular weight Mn: 3, 500, Mw/Mn ratio: 2.3; manufactured by Wako Pure Chemical Industries Ltd.) (6.2733 g) was dissolved in dichloromethane (11.01 g). This solution (13.23514 g) was weighed and blended with a solution prepared by dissolving an acetate of compound (I) (1.3694 g) in methanol (5.77 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to obtain an O/W emulsion (evolution of turbine: about 7, 000 rpm). The O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMAC CR 5DL, manufactured by Hitachi, Ltd). This was dispersed again in distilled water and further centrifuged to wash away a free medicine, etc. The collected microspheres were redispersed in a small amount of distilled water and mannitol (0.852 g) was added. The mixture was lyophilized by a lyophilizer (DF-01H, ULVAC) to obtain microcapsule powder. The content of compound (I) in the resulting microcapsule powder was 15.6%.

### Example 31

A lactic acid-glycolic acid copolymer (lactic acid/glycolic acid = 95/5, weight average  molecular weight Mw: 10, 100, number average molecular weight Mn: 4, 100, Mw/Mn ratio: 2.5; manufactured by Wako Pure Chemical Industries Ltd.) (6.2776 g) was dissolved in dichloromethane (11.10 g). This solution (13.23086 g) was weighed and blended with a solution prepared by dissolving an acetate of compound (I) (1.3706 g) in methanol (5.67 g) to obtain an oil phase. Subsequently, the oil phase was poured in a 0.1% (w/w) aqueous polyvinyl alcohol (EG-40, manufactured by Nippon Synthetic Chemical Industry Co., Ltd) solution (1 liter), which was previously adjusted to about 18°C, and emulsified by use of a turbine-form homomixer (manufactured by Tokushukika) to obtain an O/W emulsion (evolution of turbine: about 7, 000 rpm). The O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.855 g) was added. The mixture was lyophilized by a lyophilizer (DF-01H, ULVAC) to obtain microcapsule powder. The content of compound (I) in the resulting microcapsule powder was 15.0%.

### Example 32

A lactic acid-glycolic acid copolymer (lactic acid/glycolic acid = 95/5, weight average molecular weight Mw: 12, 200, number average molecular weight Mn: 4, 900, Mw/Mn ratio: 2.5; manufactured by Wako Pure Chemical Industries Ltd.) (6.2711 g) was dissolved in dichloromethane (11.01 g). This solution (13.23944 g) was weighed and blended with a solution prepared by dissolving an acetate of compound (I) (1.3664 g) in methanol (5.66 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to obtain an O/W emulsion (evolution of turbine: about 7, 000 rpm). The O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.850 g) was added. The mixture was lyophilized by a lyophilizer (DF-01H, ULVAC) to obtain microcapsule powder. The content of compound (I) in the resulting microcapsule powder was 14.3%.

### Example 33

A lactic acid-glycolic acid copolymer (lactic acid/glycolic acid = 95/5, weight average molecular weight Mw: 14, 000, number average molecular weight Mn: 5, 900, Mw/Mn ratio: 2.4; manufactured by Wako Pure Chemical Industries Ltd.) (13.2578 g) was dissolved in dichloromethane (24.09 g). This solution (13.24 g) was weighed and blended with a solution prepared by dissolving an acetate of compound (I) (1.4783g) in methanol (5.62 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to obtain an O/W emulsion (evolution of turbine: about 7, 000 rpm). The O/W emulsion was stirred for about 3 hours (in-water-drying method step) and sieved by use of a 75

μm standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.854 g) was added. The mixture was lyophilized by a lyophilizer (DF-01H, ULVAC) to obtain microcapsule powder. The content of compound (I) in the resulting microcapsule powder was 15.7%.

**Example 34**

A lactic acid-glycolic acid copolymer (lactic acid/glycolic acid = 95/5, weight average molecular weight Mw: 16, 300, number average molecular weight Mn: 6, 500, Mw/Mn ratio: 2.5; manufactured by Wako Pure Chemical Industries Ltd.) (13.2583 g) was dissolved in dichloromethane (24.07 g). This solution (13.21 g) was weighed and blended with a solution prepared by dissolving an acetate of compound (I) (1.4795g) in methanol (5.60 g) to obtain an oil phase. Subsequently, the oil phase was poured in a 0.1% (w/w) aqueous polyvinyl alcohol (EG-40, manufactured by Nippon Synthetic Chemical Industry Co., Ltd) solution (1 liter), which was previously adjusted to about 18°C, and emulsified by use of a turbine-form homomixer (manufactured by Tokushukika) to obtain an O/W emulsion (evolution of turbine: about 7, 000 rpm). The O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.864 g) was added. The mixture was lyophilized by a lyophilizer (DF-01H, ULVAC) to obtain microcapsule powder. The content of compound (I) in the resulting microcapsule powder was 16.6%.

**Example 35**

A lactic acid-glycolic acid copolymer (lactic acid/glycolic acid = 95/5, weight average molecular weight Mw: 18, 300, number average molecular weight Mn: 6, 900, Mw/Mn ratio: 2.7; manufactured by Wako Pure Chemical Industries Ltd.) (13.2370 g) was dissolved in dichloromethane (24.08 g). This solution (13.24 g) was weighed and blended with an aqueous solution prepared by dissolving an acetate of compound (I) (1.4826g) in methanol (5.62 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to obtain an O/W emulsion (evolution of turbine: about 7, 000 rpm). The O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.852 g) was added. The mixture was lyophilized by a lyophilizer (DF-01H, ULVAC) to obtain microcapsule powder. The content of compound (I) in the resulting microcapsule powder was 16.5%.

**Example 36**

A lactic acid-glycolic acid copolymer (lactic acid/glycolic acid = 95/5, weight average molecular weight Mw: 12, 200, number average molecular weight Mn: 4, 900, Mw/Mn ratio: 2.5; manufactured by Wako Pure Chemical Industries Ltd.) (13.26412 g) was dissolved in dichloromethane (24.13 g). This solution (13.31 g) was weighed, blended with an aqueous solution prepared by dissolving an acetate of compound (I) (1.48118 g) in distilled water (1.20759 g) and emulsified by use of a small homogenizer (KINEMATICA) to form a W/O emulsion (rotation number: about 10, 000 rpm, 30 sec). Subsequently, the W/O emulsion, which was adjusted to 32°C, was poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMAC CR 5DL, manufactured by Hitachi, Ltd). This was dispersed again in distilled water and further centrifuged to wash away a free medicine, etc. The collected microspheres were redispersed in a small amount of distilled water and mannitol (0.890 g) was added. The mixture was lyophilized by a lyophilizer (DF-01H, ULVAC) to obtain microcapsule powder. The content of compound (I) in the resulting microcapsule powder was 11.3%.

**Example 37**

A lactic acid-glycolic acid copolymer (lactic acid/glycolic acid = 95/5, weight average molecular weight Mw: 14, 000, number average molecular weight Mn: 5, 900, Mw/Mn ratio: 2.4; manufactured by Wako Pure Chemical Industries Ltd.) (13.28203 g) was dissolved in dichloromethane (24.08 g). This solution (13.39 g) was weighed, blended with an aqueous solution prepared by dissolving an acetate of compound (I) (1.45645 g) in distilled water (1.20538 g) and emulsified by use of a small homogenizer (KINEMATICA) to form a W/O emulsion (rotation number: about 10, 000 rpm, 30 sec). Subsequently, the W/O emulsion, which was adjusted to 32°C, was poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.909 g) was added. The mixture was lyophilized by a lyophilizer (DF-01H, ULVAC) to obtain microcapsule powder. The content of compound (I) in the resulting microcapsule powder was 12.4%.

**Example 38**

A lactic acid-glycolic acid copolymer (lactic acid/glycolic acid = 95/5, weight average molecular weight Mw: 16, 300, number average molecular weight Mn: 6, 500, Mw/Mn ratio: 2.5; manufactured by Wako Pure Chemical Industries Ltd.) (13.25986 g) was dissolved in dichloromethane (24.05 g). This solution (13.23 g) was weighed, blended with an aqueous solution prepared by dissolving an acetate of compound (I) (1.48385 g) in distilled water (1.20734 g) and emulsified by use of a small homogenizer (KINEMATICA) to form a W/O emulsion (rotation number: about 10, 000 rpm, 30 sec). Subsequently, the W/O emulsion, which was adjusted to 32°C, was poured in a 0.1% (w/w) aqueous polyvinyl alcohol (EG-40, manufactured by Nippon Synthetic Chemical Industry Co., Ltd) solution (1 liter), which was previously adjusted to about 18°C, and subjected to secondary emulsification using a turbine-form homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.886 g) was added. The mixture was lyophilized by a lyophilizer (DF-01H, ULVAC) to obtain microcapsule powder. The content of compound (I) in the resulting microcapsule powder was 10.9%.

**Example 39**

A lactic acid-glycolic acid copolymer (lactic acid/glycolic acid = 95/5, weight average molecular weight Mw: 18, 300, number average molecular weight Mn: 6, 900, Mw/Mn ratio: 2.7; manufactured by Wako Pure Chemical Industries Ltd.) (13.27697 g) was dissolved in dichloromethane (24.03 g). This solution (13.28 g) was weighed, blended with an aqueous solution prepared by dissolving an acetate of compound (I) (1.47734 g) in distilled water (1.20452 g) and emulsified by use of a small homogenizer (KINEMATICA) to form a W/O emulsion (rotation number: about 10, 000 rpm, 30 sec). Subsequently, the W/O emulsion, which was adjusted to 32°C, was poured in a 0.1% (w/w) aqueous polyvinyl alcohol (EG-40, manufactured by Nippon Synthetic Chemical Industry Co., Ltd.) solution (1 liter), which was previously adjusted to about 18°C, and subjected to secondary emulsification using a turbine-form homomixer (manufactured by Tokushukika) to prepare a W/O/W emulsion (evolution of turbine: about 7, 000 rpm). The W/O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.878 g) was added. The mixture was lyophilized by a lyophilizer (DF-01H, ULVAC) to obtain microcapsule powder. The content of compound (I) in the resulting microcapsule powder was 10.9%.

**Example 40**

A lactic acid-glycolic acid copolymer (lactic acid/glycolic acid = 85/15, weight average molecular weight Mw: 16, 200, number average molecular weight Mn: 6, 800, Mw/Mn ratio: 2.4; manufactured by Wako Pure Chemical Industries Ltd.) (7.020 g) was dissolved in dichloromethane (13.132 g). This solution (15.4976 g) was weighed and blended with a solution prepared by dissolving an acetate of compound (I) (0.6831 g) in methanol (2.79729g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7, 000 rpm). The O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMAC CR 5DL, manufactured by Hitachi, Ltd). This was dispersed again in distilled water and further centrifuged to wash away a free medicine, etc. The collected microspheres were redispersed in a small amount of distilled water and mannitol (0.842 g) was added. The mixture was lyophilized by a lyophilizer (DF-01H, ULVAC) to obtain microcapsule powder. The content of compound (I) in the resulting microcapsule powder was 7.7%.

## Example 41

A lactic acid-glycolic acid copolymer (lactic acid/glycolic acid = 85/15, weight average molecular weight Mw: 18, 100, number average molecular weight Mn: 7, 500, Mw/Mn ratio: 2.4; manufactured by Wako Pure Chemical Industries Ltd.) (7.022 g) was dissolved in dichloromethane (13.132 g). This solution (15.4936 g) was weighed and blended with a solution prepared by dissolving an acetate of compound (I) (0.6831 g) in methanol (2.8081 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7, 000 rpm). The O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.842 g) was added. The mixture was lyophilized by a lyophilizer (DF-01H, ULVAC) to obtain microcapsule powder. The content of compound (I) in the resulting microcapsule powder was 8.2%.

## Example 42

A lactic acid-glycolic acid copolymer (lactic acid/glycolic acid = 85/15, weight average molecular weight Mw: 16, 200, number average molecular weight Mn: 6, 800, Mw/Mn ratio: 2.4; manufactured by Wako Pure Chemical Industries Ltd.) (6.243 g) was dissolved in dichloromethane (10.924 g). This solution (13.2289 g) was weighed and blended with a solution prepared by dissolving an acetate of compound (I) (1.3647 g) in methanol (5.6052 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7, 000 rpm). The O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.856 g) was added. The mixture was lyophilized by a lyophilizer (DF-01H, ULVAC) to obtain microcapsule powder. The content of compound (I) in the resulting microcapsule powder was 14.9%.

## Example 43

A lactic acid-glycolic acid copolymer (lactic acid/glycolic acid = 85/15, weight average molecular weight Mw: 18, 100, number average molecular weight Mn: 7, 500, Mw/Mn ratio: 2.4; manufactured by Wako Pure Chemical Industries Ltd.) (6.243 g) was dissolved in dichloromethane (10.921 g). This solution (113.2253 g) was weighed and blended with a solution prepared by dissolving an acetate of compound (I) (1.3668 g) in methanol (5.6024 g) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7, 000 rpm). The O/W emulsion was stirred for about 3 hours (in-water-drying method step) and sieved by use of a 75 μm standard sieve, and then, microspheres were collected by centrifugation (rotation number: about 2, 500 rpm, 5 min) using a centrifuge (HIMAC CR 5DL, manufactured by Hitachi, Ltd). This was dispersed again in distilled water and further centrifuged to wash away a free medicine, etc. The collected microspheres were redispersed in a small amount of distilled water and mannitol (0.842 g) was added. The mixture was lyophilized by a lyophilizer (DF-01H, ULVAC) to obtain microcapsule powder. The content of compound (I) in the resulting microcapsule powder was 14.1%.

### Example 44

Acetic acid (6.92642 g) and methanol (4.82027 g) were blended. The resulting solution (4.76611 g) was used for dissolving tryptophane (0.26503 g). The methanol/acetic acid solution dissolving tryptophane therein was used for dissolving an acetate of compound (I) (1.21857 g) to prepare a methanol/acetic acid solution in which tryptophane and the acetate of compound (I) were dissolved.

A lactic acid-glycolic acid copolymer (lactic acid/glycolic acid = 95/5, weight average molecular weight Mw: 10, 100, number average molecular weight Mn: 4, 100, Mw/Mn ratio: 2.5; manufactured by Wako Pure Chemical Industries Ltd.) (6.26043 g) was dissolved in dichloromethane (11.04 g). This solution (13.39 g) was weighed and blended with the methanol/acetic acid solution dissolving tryptophane and the acetate of compound (I) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7, 000 rpm). The O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.849 g) was added. The mixture was lyophilized by a lyophilizer (DF-01H, ULVAC) to obtain microcapsule powder. The content of compound (I) in the resulting microcapsule powder was 13.2%.

### Example 45

Acetic acid (6.91984 g) and methanol (4.79744 g) were blended. The resulting solution (4.63624 g) was used for dissolving tryptophane (0.13000 g). The methanol/acetic acid solution dissolving tryptophane was used for dissolving an acetate of compound (I) (1.20032 g) to prepare a methanol/acetic acid solution in which tryptophane and the acetate of compound (I) were dissolved.

A lactic acid-glycolic acid copolymer (lactic acid/glycolic acid = 95/5, weight average molecular weight Mw: 10, 100, number average molecular weight Mn: 4, 100, Mw/Mn ratio: 2.5; manufactured by Wako Pure Chemical Industries Ltd.) (6.25830 g) was dissolved in dichloromethane (11.06 g). This solution (13.40 g) was weighed and blended with the methanol/acetic acid solution dissolving tryptophane and the acetate of compound (I) to obtain an oil phase. Subsequently, the oil phase was poured in a 0.1% (w/w) aqueous polyvinyl alcohol (EG-40, manufactured by Nippon Synthetic Chemical Industry Co., Ltd) solution (1 liter), which was previously adjusted to about 18°C, and emulsified by use of a turbine-form homomixer (manufactured by Tokushukika) to prepare an O/W emulsion (evolution of turbine: about 7, 000 rpm). The O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.217 g) was added. The mixture was lyophilized by a lyophilizer (DF-01H, ULVAC) to obtain microcapsule powder.

### Example 46

Acetic acid (6.91984 g) and methanol (4.82027 g) were blended. The resulting solution (4.63624 g) was used for dissolving tryptophane (0.26503 g). The methanol/acetic acid solution dissolving tryptophane was used for dissolving an acetate of compound (I) (1.21100 g) to prepare a methanol/acetic acid solution dissolving tryptophane and the acetate of compound (I).

A lactic acid-glycolic acid copolymer (lactic acid/glycolic acid = 95/5, weight average molecular weight Mw: 14, 000, number average molecular weight Mn: 5, 900, Mw/Mn ratio: 2.4; manufactured by Wako Pure Chemical Industries Ltd.) (6.26571 g) was dissolved in dichloromethane (11.01 g). This solution (13.35 g) was weighed and blended with the methanol/acetic acid solution dissolving tryptophane and the acetate of compound (I) to obtain an oil phase. Subsequently, the oil phase was poured in a 0.1% (w/w) aqueous polyvinyl alcohol (EG-40, manufactured by Nippon Synthetic Chemical Industry Co., Ltd.) solution (1 liter), which was previously adjusted to about 18°C, and emulsified by use of a turbine-form homomixer (manufactured by Tokushukika) to prepare an O/W emulsion (evolution of turbine: about 7, 000 rpm). The O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.849 g) was added. The mixture was lyophilized by a lyophilizer (DF-01H, ULVAC) to obtain microcapsule powder.

**Example 47**

Acetic acid (6.91984 g) and methanol (4.82027 g) were blended. The resulting solution (4.63624 g) was used for dissolving tryptophane (0.13000 g). The methanol/acetic acid solution dissolving tryptophane was used for dissolving an acetate of compound (I) (1.20702 g) to prepare a methanol/acetic acid solution in which tryptophane and the acetate of compound (I) were dissolved.

A lactic acid-glycolic acid copolymer (lactic acid/glycolic acid = 95/5, weight average molecular weight Mw: 14, 000, number average molecular weight Mn: 5, 900, Mw/Mn ratio: 2.4; manufactured by Wako Pure Chemical Industries Ltd.) (6.27641 g) was dissolved in dichloromethane (10.96 g). This solution (13.32 g) was weighed and blended with the methanol/acetic acid solution dissolving tryptophane and the acetate of compound (I) to obtain an oil phase. Subsequently, the oil phase was poured in a 0.1% (w/w) aqueous polyvinyl alcohol (EG-40, manufactured by Nippon Synthetic Chemical Industry Co., Ltd) solution (1 liter), which was previously adjusted to about 18°C, and emulsified by use of a turbine-form homomixer (manufactured by Tokushukika) to prepare an O/W emulsion (evolution of turbine: about 7, 000 rpm). The O/W emulsion was stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMAC CR 5DL, manufactured by Hitachi, Ltd.). This was dispersed again in distilled water and further centrifuged to wash away a free medicine, etc. The collected microspheres were redispersed in a small amount of distilled water and mannitol (0.847 g) was added. The mixture was lyophilized by a lyophilizer (DF-01H, ULVAC) to obtain microcapsule powder.

**Comparative Example 1**

Lactic acid-glycolic acid copolymers (lactic acid/glycolic acid = 75/25, weight average molecular weight Mw: 4, 200, number average molecular weight Mn: 1, 800, Mw/Mn ratio: 2.3; manufactured by Wako Pure Chemical Industries Ltd.) (5.9212 g, 5.9255 g, 5.9255 g, 5.9229 g) were respectively dissolved in dichloromethane (10.9736 g, 10.9566 g, 11.0430 g, 10.9739 g). These solutions (13.1036 g, 12.9562 g, 12.9643 g, 12.9602 g) were separately weighed, blended with aqueous solutions prepared by respectively dissolving an acetate of compound (I) (0.5744 g, 0.5767 g, 0.5701 g, 0.5725 g) in distilled water (0.5017 g, 0.5356 g, 0.5119 g, 0.5203 g), and emulsified by use of a small homogenizer (KINEMATICA) to form W/O emulsions (rotation number: about 10, 000 rpm, 30 sec). Subsequently, the W/O emulsions were respectively poured in four 0.1% (w/w) aqueous polyvinyl alcohol (EG-40, manufactured by Nippon Synthetic Chemical Industry Co., Ltd.) solutions (1 liter), which were previously adjusted to about 18°C, and subjected to secondary emulsification using a turbine-form homomixer (manufactured by Tokushukika) to prepare W/O/W emulsions (evolution of turbine: about 7, 000 rpm). The W/O/W emulsions each were stirred for about 3 hours (in-water-drying method step) and sieved by use of a 75 $\mu$m standard sieve, and then, microspheres were collected by centrifugation (rotation number: about 2, 500 rpm, 5 min) using a centrifuge (HIMACCR 5DL, manufactured by Hitachi, Ltd.). This was dispersed again in distilled water and further centrifuged to wash away a free medicine, etc. The collected microspheres were all redispersed in a small amount of distilled water to put together into a whole and mannitol (2.591 g) was added. The mixture was lyophilized by a lyophilizer (DF-01H, ULVAC) to obtain microcapsule powder. The content of compound (I) in the resulting microcapsule powder was 7.2%.

**Comparative Example 2**

In the case where a solution mixture of a solution prepared by dissolving a lactic acid-glycolic acid copolymer in dichloromethane in the same manner as in Example 10 and an aqueous solution prepared by dissolving an acetate of compound No. 550 described in WO06/1499 (Ac-D-Tyr-D-Trp-Asn-Thr-Phe-AzaGly-Leu-Arg(Me) -Trp-NH$_2$) (SEQ ID NO:2), in distilled water, is allowed to suspend by a mini-mixer, the solution mixture is gelatinized. Therefore, a W/O emulsion cannot be obtained.

**Test Example 1**

The microcapsule powder (1.6 mg as a free compound (I)) of Example 1 was dispersed in a dispersion medium (0.15 mL) (a solution in which carboxymethylcellulose (0.75 mg), polysorbate 80 (0.15 mg) and mannitol (7.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 7 weeks.

**Test Example 2**

The microcapsule powder (1.6 mg as a free compound (I)) of Example 2 was dispersed in a dispersion medium (0.15 mL) (a solution in which carboxymethylcellulose (0.75 mg), polysorbate 80 (0.15 mg) and mannitol (7.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 8 weeks.

**Test Example 3**

The microcapsule powder (1.6 mg as a free compound (I)) of Example 3 was dispersed in a dispersion medium (0.15 mL) (a solution in which carboxymethylcellulose (0.75 mg), polysorbate 80 (0.15 mg) and mannitol (7.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 6 weeks.

**Test Example 4**

The microcapsule powder (1.6 mg as a free compound (I)) of Example 4 was dispersed in a dispersion medium (0.15 mL) (a solution in which carboxymethylcellulose (0.75 mg), polysorbate 80 (0.15 mg) and mannitol (7.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 5 weeks.

**Test Example 5**

The microcapsule powder (1.6 mg as a free compound (I) of Example 5 was dispersed in a dispersion medium (0.15 mL) (a solution in which carboxymethylcellulose (0.75 mg), polysorbate 80 (0.15 mg) and mannitol (7.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 5 weeks.

**Test Example 6**

The microcapsule powder (1.6 mg as a free compound (I) of Example 6 was dispersed in a dispersion medium (0.15 mL) (a solution in which carboxymethylcellulose (0.75 mg), polysorbate 80 (0.15 mg) and mannitol (7.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 3 weeks.

**Test Example 7**

The microcapsule powder (1.6 mg as a free compound (I)) of Example 7 was dispersed in a dispersion medium (0.15 mL) (a solution in which carboxymethylcellulose (0.75 mg),  polysorbate 80 (0.15 mg) and mannitol (7.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 3 weeks.

**Test Example 8**

The microcapsule powder (1.6 mg as a free compound (I)) of Example 8 was dispersed in a dispersion medium (0.15 mL) (a solution in which carboxymethylcellulose (0.75 mg), polysorbate 80 (0.15 mg) and mannitol (7.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 5 weeks.

**Test Example 9**

The microcapsule powder (1.6 mg as a free compound (I)) of Example 9 was dispersed in a dispersion medium (0.15 mL) (a solution in which carboxymethylcellulose (0.75 mg), polysorbate 80 (0.15 mg) and mannitol (7.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 6 weeks.

**Test Example 10**

The microcapsule powder (1.6 mg as a free compound (I)) of Example 10 was dispersed in a dispersion medium (0.15 mL) (a solution in which carboxymethylcellulose (0.75 mg), polysorbate 80 (0.15 mg) and mannitol (7.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 5 weeks.

**Test Example 11**

The microcapsule powder (1.6 mg as a free compound (I)) of Example 11 was dispersed in a dispersion medium (0.15 mL) (a solution in which carboxymethylcellulose (0.75 mg), polysorbate 80 (0.15 mg) and mannitol (7.5 mg) were dissolved) and subcutaneously administered to the dorsal portions of rats by means of a 22G injection needle. In a predetermined time internal after the administration, blood was sampled from the caudal vein and the concentration of compound (I) in the plasma was measured. As a result, sustained release of compound (I) was confirmed for about 5 weeks.

**Test Example 11-2**

The microcapsule powder (1.6 mg as a free compound (I)) of Example 11-2 was dispersed in a dispersion medium (0.15 mL) (a solution in which carboxymethylcellulose (0.75 mg), polysorbate 80 (0.15 mg) and mannitol (7.5 mg) were dissolved) and subcutaneously administered to the dorsal portions of rats by means of a 22G injection needle. In a predetermined time interval after the administration, blood was sampled from the causal vein and the concentration of compound (I) in the plasma was measured. As a result, sustained release of compound (I) was confirmed for about 5 weeks.

**Test Example 12**

The microcapsule powder (4.8 mg as a free compound (I)) of Example 12 was dispersed in a dispersion medium (0.45 mL) (a solution in which carboxymethylcellulose (2.25 mg), polysorbate 80 (0.45 mg) and mannitol (22.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 12 weeks.

**Test Example 13**

The microcapsule powder (4.8 mg as a free compound (I)) of Example 13 was dispersed in a dispersion medium (0.45 mL) (a solution in which carboxymethylcellulose (2.25 mg), polysorbate 80 (0.45 mg) and mannitol (22.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 16 weeks.

**Test Example 14**

The microcapsule powder (4.8 mg as a free compound (I)) of Example 14 was dispersed in a dispersion medium (0.45 mL) (a solution in which carboxymethylcellulose (2.25 mg), polysorbate 80 (0.45 mg) and mannitol (22.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of  compound (I) was confirmed for about 14 weeks.

**Test Example 15**

The microcapsule powder (4.8 mg as a free compound (I)) of Example 15 was dispersed in a dispersion medium (0.45 mL) (a solution in which carboxymethylcellulose (2.25 mg), polysorbate 80 (0.45 mg) and mannitol (22.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 12 weeks.

**Test Example 16**

The microcapsule powder (4.8 mg as a free compound (I)) of Example 16 was dispersed in a dispersion medium (0.45 mL) (a solution in which carboxymethylcellulose (2.25 mg), polysorbate 80 (0.45 mg) and mannitol (22.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 13 weeks.

**Test Example 17**

The microcapsule powder (4.8 mg as a free compound (I)) of Example 17 was dispersed in a dispersion medium (0.45 mL) (a solution in which carboxymethylcellulose (2.25 mg), polysorbate 80 (0.45 mg) and mannitol (22.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 12 weeks.

**Test Example 18**

The microcapsule powder (4.8 mg as a free compound (I)) of Example 18 was dispersed in a dispersion medium (0.45 mL) (a solution in which carboxymethylcellulose (2.25 mg), polysorbate 80 (0.45 mg) and mannitol (22.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 13 weeks.

**Test Example 19**

The microcapsule powder (4.8 mg as a free compound (I)) of Example 19 was dispersed in a dispersion medium (0.45 mL) (a solution in which carboxymethylcellulose (2.25 mg), polysorbate 80 (0.45 mg) and mannitol (22.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 13 weeks.

**Test Example 20**

The microcapsule powder (4.8 mg as a free compound (I)) of Example 20 was dispersed in a dispersion medium (0.45 mL) (a solution in which carboxymethylcellulose (2.25 mg), polysorbate 80 (0.45 mg) and mannitol (22.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (1) was confirmed for about 14 weeks.

**Test Example 21**

The microcapsule powder (1.6 mg as a free compound (I) of Comparative Example 1 was dispersed in a dispersion medium (0.15 mL) (a solution in which carboxymethylcellulose (0.75 mg), polysorbate 80 (0.15 mg) and mannitol (7.5 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 2 weeks.

**Test Example 22**

The microcapsule powder (4.8 mg as a free compound (I)) of Example 25 was dispersed in a dispersion medium (0.45 mL) (a solution in which carboxymethylcellulose (2.25 mg), polysorbate 80 (0.45 mg) and mannitol (2.25 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 15 weeks.

**Test Example 23**

The microcapsule powder (4.8 mg as a free compound (I)) of Example 27 was dispersed in a dispersion medium (0.45 mL) (a solution in which carboxymethylcellulose (2.25 mg), polysorbate 80 (0.45 mg) and mannitol (2.25 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 18 weeks.

**Test Example 24**

The microcapsule powder (4.8 mg as a free compound (I)) of Example 29 was dispersed in a dispersion medium (0.45 mL) (a solution in which carboxymethylcellulose (2.25 mg), polysorbate 80 (0.45 mg) and mannitol (2.25 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 18 weeks.

**Test Example 25**

The microcapsule powder (4.8 mg as a free compound (I)) of Example 31 was dispersed in a dispersion medium (0.45 mL) (a solution in which carboxymethylcellulose (2.25 mg), polysorbate 80 (0.45 mg) and mannitol (2.25 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 18 weeks.

**Test Example 26**

The microcapsule powder (4.8 mg as a free compound (I)) of Example 32 was dispersed in a dispersion medium (0.45 mL) (a solution in which carboxymethylcellulose (2.25 mg), polysorbate 80 (0.45 mg) and mannitol (2.25 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 15 weeks.

**Test Example 27**

The microcapsule powder (4.8 mg as a free compound (I)) of Example 33 was dispersed in a dispersion medium (0.45 mL) (a solution in which carboxymethylcellulose (2.25 mg), polysorbate 80 (0.45 mg) and mannitol (2.25 mg) were dissolved) and subcutaneously administered to the dorsal portions of rats by means of a 22G injection needle. In a  predetermined time interval after the administration, blood was sampled from the caudal vein and the concentration of compound (I) in the plasma was measured. As a result, sustained release of compound (I) was confirmed for about 15 weeks.

**Test Example 28**

The microcapsule powder (4.8 mg as a free compound (I)) of Example 35 was dispersed in a dispersion medium (0.45 mL) (a solution in which carboxymethylcellulose (2.25 mg), polysorbate 80 (0.45 mg) and mannitol (2.25 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 15 weeks.

**Test Example 29**

The microcapsule powder (4.8 mg as a free compound (I)) of Example 37 was dispersed in a dispersion medium (0.45 mL) (a solution in which carboxymethylcellulose (2.25 mg), polysorbate 80 (0.45 mg) and mannitol (2.25 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 18 weeks.

**Test Example 30**

The microcapsule powder (4.8 mg as a free compound (I)) of Example 39 was dispersed in a dispersion medium (0.45 mL) (a solution in which carboxymethylcellulose (2.25 mg), polysorbate 80 (0.45 mg) and mannitol (2.25 mg) were dissolved) and subcutaneously administered to the dorsal portions of rats by means of a 22G injection needle. In a predetermined time interval after the administration, blood was sampled from the caudal vein and the concentration of compound (I) in the plasma was measured. As a result, sustained release of compound (I) was confirmed for about 18 weeks.

**INDUSTRIAL APPLICABILITY**

The sustained-release preparation of the present invention stably releases a metastin derivative for a long time and also exerts the efficacy of the metastin derivative for a long time. Furthermore, the sustained-release preparation of the present invention can reduce the administration frequency, thereby improving convenience of patients and can be used as a clinical medicine.

**Claims**

1. A sustained-release formulation comprising a compound represented by Formula:

   Ac-D-Tyr-Hyp-Asn-Thr-Phe-AzaGly-Leu-Arg(Me) -Trp-NH$_2$ (I)

   or a salt thereof and a lactic acid-glycolic acid copolymer having a weight average molecular weight of 5,000 to 40,000 or a salt thereof

2. The sustained-release formulation according to claim 1, wherein the weight average molecular weight of the lactic acid-glycolic acid copolymer is 6,000 to 20,000.

3. The sustained-release formulation according to claim 1, wherein the content of a glycolic acid of the lactic acid-glycolic acid copolymer or a salt thereof is greater than 0 wt% and 60 wt% or less.

4. The sustained-release formulation according to claim 1, wherein the content of a glycolic acid of the lactic acid-glycolic acid copolymer is 5 wt% or more and 50 wt% or less.

5. The sustained-release formulation according to claim 1, wherein the formulation is a therapeutic or prophylactic agent for cancer.

6. The sustained-release formulation according to claim 1, wherein the formulation is a parenteral agent.

7. A method for producing the sustained-release formulation according to claim 1, comprising producing a W/O emulsion composed of an internal water phase, which contains a compound represented by Formula:

   Ac-D-Tyr-Hyp-Asn-Thr-Phe-AzaGly-Leu-Arg(Me) -Trp-NH$_2$ (I)

   or a salt thereof, and an oil phase, which contains a lactic acid-glycolic acid copolymer or a salt thereof, further emulsifying the W/O emulsion to obtain a W/O/W emulsion, and subjecting the W/O/W emulsion to an in-water-drying method.

8. The method according to claim 7, wherein the W/O emulsion is produced at a temperature of 31°C or more.

**Patentansprüche**

1. Zubereitung mit nachhaltiger Wirkstofffreisetzung, umfassend eine Verbindung, die durch die Formel

Ac-D-Tyr-Hyp-Asn-Thr-Phe-AzaGly-Leu-Arg(Me) -Trp-NH$_2$ (1)

dargestellt wird, oder ein Salz davon und ein Milchsäure-Glycolsäure-Copolymer mit einem Gewichtsmittel des Molekulargewichts von 5000 bis 40 000 oder ein Salz davon.

**2.** Zubereitung mit nachhaltiger Wirkstofffreisetzung gemäß Anspruch 1, wobei das Gewichtsmittel des Molekulargewichts des Milchsäure-Glycolsäure-Copolymers 6000 bis 20 000 beträgt.

**3.** Zubereitung mit nachhaltiger Wirkstofffreisetzung gemäß Anspruch 1, wobei der Gehalt der Glycolsäure in dem Milchsäure-Glycolsäure-Copolymer oder einem Salz davon größer als 0 Gew.-% ist und 60 Gew.-% oder weniger beträgt.

**4.** Zubereitung mit nachhaltiger Wirkstofffreisetzung gemäß Anspruch 1, wobei der Gehalt der Glycolsäure in dem Milchsäure-Glycolsäure-Copolymer 5 Gew.-% oder mehr und 50 Gew.-% oder weniger beträgt.

**5.** Zubereitung mit nachhaltiger Wirkstofffreisetzung gemäß Anspruch 1, wobei die Zubereitung ein therapeutisches oder prophylaktisches Mittel gegen Krebs ist.

**6.** Zubereitung mit nachhaltiger Wirkstofffreisetzung gemäß Anspruch 1, wobei die Zubereitung ein parenterales Mittel ist.

**7.** Verfahren zur Herstellung der Zubereitung mit nachhaltiger Wirkstofffreisetzung gemäß Anspruch 1, umfassend das Herstellen einer W/O-Emulsion, die aus einer internen Wasserphase, die eine durch die Forme

Ac-D-Tyr-Hyp-Asn-Thr-Phe-AzaGly-Leu-Arg(Me) -Trp-NH$_2$ (1)

dargestellte Verbindung oder ein Salz davon enthält, und einer Ölphase, die ein Milchsäure-Glycolsäure-Copolymer oder ein Salz davon enthält, besteht, weiterhin das Emulgieren der W/O-Emulsion, wobei man eine W/O/W-Emulsion erhält, und das Behandeln der W/O/W-Emulsion durch ein In-Wasser-Trocknungsverfahren.

**8.** Verfahren gemäß Anspruch 7, wobei die W/O-Emulsion bei einer Temperatur von 31°C oder mehr hergestellt wird.


**Revendications**

**1.** Formulation à libération entretenue, comprenant un composé représenté par la formule

Ac-D-Tyr-Hyp-Asn-Thr-Phe-AzaGly-Leu-Arg(Me) -Trp-NH$_2$ (I)

ou un sel de ce composé, et un copolymère d'acide lactique et d'acide glycolique dont la masse molaire moyenne en poids vaut de 5 000 à 40 000 ou un sel d'un tel copolymère.

**2.** Formulation à libération entretenue, conforme à la revendication 1, dans laquelle la masse molaire moyenne en poids du copolymère d'acide lactique et d'acide glycolique vaut de 6 000 à 20 000.

**3.** Formulation à libération entretenue, conforme à la revendication 1, dans laquelle la proportion de l'acide glycolique dans le copolymère d'acide lactique et d'acide glycolique ou son sel est supérieure à 0 % en poids, mais inférieure ou égale à 60 % en poids.

**4.** Formulation à libération entretenue, conforme à la revendication 1, dans laquelle la proportion de l'acide glycolique dans le copolymère d'acide lactique et d'acide glycolique est supérieure ou égale à 5 % en poids, mais inférieure ou égale à 50 % en poids.

**5.** Formulation à libération entretenue, conforme à la revendication 1, laquelle formulation est un agent thérapeutique ou prophylactique contre un cancer.

**6.** Formulation à libération entretenue, conforme à la revendication 1, laquelle formulation est un agent administré par voie parentérale.

**7.** Procédé de préparation d'une formulation à libération entretenue, conforme à la revendication 1, lequel procédé comprend le fait de préparer une émulsion de type eau-dans-huile, constituée d'une phase interne aqueuse, qui contient un composé représenté par la formule

Ac-D-Tyr-Hyp-Asn-Thr-Phe-AzaGly-Leu-Arg(Me) -Trp-NH$_2$ (I)

ou un sel de ce composé, et d'une phase huileuse qui contient un copolymère d'acide lactique et d'acide glycolique ou un sel d'un tel copolymère, puis le fait d'émulsifier cette émulsion eau-dans-huile de manière à obtenir une émulsion eau-dans-huile-dans-eau, et le fait de soumettre cette émulsion eau-dans-huile-dans-eau à une opération de séchage dans de l'eau.

**8.** Procédé conforme à la revendication 7, dans lequel on prépare l'émulsion eau-dans-huile à une température égale ou supérieure à 31 °C.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0772997 A
- WO 0285399 A
- WO 061499 A
- JP 2009290364 A
- JP 2010144793 A

**Non-patent literature cited in the description**

- *Cancer Research,* 1989, vol. 49, 5935-5939
- *Journal of Clinical Oncology,* 1994, vol. 12, 213-225
- *British Journal of Cancer,* 1993, vol. 68, 314-318